# EUROPEAN PATENT SPECIFICATION

(11) **EP 2 213 327 B1**
(45) Date of publication and mention of the grant of the patent: **30.10.2013**
(21) Application number: 08833128.5
(22) Date of filing: 25.09.2008
(51) Int. Cl.: A61M 39/00, A61M 25/00, A61M 25/08, A61M 39/06, F16K 15/18, F16K 15/14, A61M 39/24, A61M 25/06

(54) **VALVE ELEMENT AND MEDICAL INSTRUMENT**
VENTILELEMENT UND MEDIZINISCHES INSTRUMENT
ÉLÉMENT DE SOUPAPE ET INSTRUMENT MÉDICAL

(30) Priority: 27.09.2007 JP 2007252478; 27.09.2007 JP 2007252479
(43) Date of publication of application: 04.08.2010
(73) Proprietor: Terumo Kabushiki Kaisha, Tokyo, 1510072 (JP)
(72) Inventor: SUGIKI, Tsutomu, Fujinomiya-shi Shizuoka 409-3853 (JP); KASAI, Masaaki, Fujinomiya-shi Shizuoka 409-3853 (JP); IMAI, Yukio, Fujinomiya-shi Shizuoka 409-3853 (JP); ISHII, Shingo, Fujinomiya-shi Shizuoka 409-3853 (JP)
(74) Representative: Prüfer & Partner GbR European Patent Attorneys
(86) International application number: PCT/JP2008/067329
(87) International publication number: WO 2009/041523

(56) References cited:
- EP-A1- 0 771 574
- EP-A2- 0 308 815
- WO-A1-2006/086711
- WO-A1-2006/086711
- WO-A2-01/54763
- WO-A2-99/06099
- JP-A- 4 170 966
- JP-A- 2004 154 456
- JP-B2- 2 510 887
- JP-T- 2003 520 652
- JP-T- 2003 522 545
- JP-Y2- 5 004 843
- US-A- 5 167 637
- US-A- 5 273 546
- US-A- 5 312 363
- US-A- 5 496 289
- US-A- 5 496 289

## Description

### Technical Field

This invention relates to a valve body and a medical tool which have an opening and closing section which is opened and closed in response to insertion and pulling off of an elongated member such as, for example, a catheter, a guide wire or a dilator.

### Background Art

When an elongated member used for medical care such as a catheter or a guide wire is to be introduced into a living organism, an introducer (medical tool) is used. As one of such introducers, an introducer disclosed, for example, in Patent Document 1 is known.

This introducer is composed of a hub having a tubular shape, a sheath having a cap mounted at one end portion of the hub and a sheath tube connected to the other end portion of the hub, and a dilator which is inserted into and used together with the sheath in order to facilitate introduction of the sheath into a living organism. An opening is provided in the cap such that it is communicated with a hollow of the hub. A valve body having a disk shape is provided so as to cover the opening, that is, so as to maintain a liquid-tight state of the hollow of the hub.

The valve body is made of an elastic material such as silicone rubber and has two slits formed thereon so as to intersect crosswise with each other. The slits are placed into an open state when an elongated member (pipe body) is inserted into the sheath, but are placed into a closed state when the elongated member is pulled off from the sheath.

However, with such a valve body as just described, when an elongated member is inserted into the valve body, an end portion of the elongated member may pierce a portion of the valve body other than the slits, resulting in damage to the slits.

Further, since inner faces contact with each other at the slits, if radiation sterilization (for example, electron beam sterilization, γ ray sterilization, X-ray sterilization or the like) is carried out, then blocking occurs between the contact faces at the slit portions. As a result, the penetration resistance when an elongated member is inserted into the valve body after the sterilization increases, and this makes it less easy to insert an elongated member. Further, upon insertion, the slits are less likely to come off and may be damaged.

If the slits are damaged, then even if the elongated member is pulled off and the slits are closed, the liquid-tightness of the valve body (slits) may be deteriorated (damaged) (this similarly applies also to a state wherein elongated member is inserted).

For example, where a small tear appears with any slit, if insertion and pulling off of an elongated member are repeated frequently, then the tear grows into a large tear, and this gives rise to such a problem as described above.

Patent Document 1: Japanese Patent Publication No. Hei 2-949 (JPS 59- 133877).
Valve bodies are known from US 5,312,363 A; JP 2004 154456 A; JP 5 004843 Y2; JP 6 039011 U; EP 0 771 574 A1; WO 2006/086711 A1; US 5,496,289 A; and WO 99/06099 A2.

The preamble of claim 1 starts out from a valve body having two slifs and an upper face with a concave partion, as e.g. shown in EP0308815.

It is the object of the present invention to provide a valve body made of an elastic material and having an opening and closing section which opens and closes in response to insertion and pulling out of an elongated member into and from said valve body and to provide a medical tool comprising the valve body, by which insertion and pulling-off of the elongated member can be carried out readily and. wherein no tear appears with the slit of the opening and closing section. This object is achieved by the valve body having the features of claim 1 and by the medical tool having the features of claim 5. The invention is further developed as defined in the dependent claims.

In order to attain the object described above, according to the present invention, there is provided a valve body made of an elastic material and having an opening and closing section which opens and closes in response to insertion and pulling out of an elongated member into and from the valve body, the valve body having a first concave portion formed on an upper face side thereof and having a bottom face formed as a concave face, the valve body further having a lower face on the opposite side to the upper face, wherein the opening and closing section includes:
a first slit extending to the concave face but not to the lower face;
a second slit extending to the lower face but not to the concave face and intersecting with the first slit in the inside of the valve body; and
a second concave portion provided at a location of the concave face side corresponding to the intersecting portion between the first slit and the second slit and extending not to the second slit.

According to the configuration, when an elongated member is inserted into the valve body (opening and closing section), the elongated member can be introduced to a portion of the first slit corresponding to the intersecting portion readily and with certainty by the first concave portion. Consequently, such a situation that any other portion than the first slit or the second slit is pierced by the elongated member and the valve body (particularly the first slit, the second slit, a portion in the proximity of the first slit or the second slit or a like portion) is damaged can be prevented. Further, the distal end portion of the elongated member can be prevented from crushed or deformed.

Further, since the second concave portion is provided, the contact area of the inner faces of the first slit decreases, and consequently, the area over which blocking occurs when radiation sterilization is carried out (area over which the inner faces stick to each other) can be reduced. Further, when an elongated member is inserted into the valve body for the first time after sterilization, the second concave portion provides a start of exfoliating of the first slit which is in a sticking state. Consequently, upon insertion of the elongated member, the first slit and the second slit exfoliate readily and damage to the first slit or the second slit can be prevented. Further, the penetration resistance upon insertion decreases and the elongated member can be inserted into the valve body readily.

Therefore, the valve body of the present invention can be used (installed) in a sheath of an introducer for introducing an elongated member for use with medical care such as, for example, a catheter or a guide wire into a living organism.

Preferably, in the valve body of the present invention, the intersecting portion is positioned at a central portion of the valve body as viewed in plan, and
the first concave portion includes the intersecting portion as viewed in plan and the intersecting portion is provided in such a manner as to be positioned at a central portion of the first concave portion while
the first slit is provided so as to extend from one end portion to the other end portion of the first concave portion.
According to the configuration, when the elongated member is inserted into and pulled off from the valve body, the operation can be carried out stably.

Preferably, in the valve body of the present invention, the first concave portion has a bowl-like shape.
According to the configuration, when the elongated member is inserted into the valve body, it can be introduced to a portion of the first slit corresponding to the intersecting portion more readily and with a higher degree of accuracy by the first concave portion.

Preferably, in the medical tool and valve body of the present invention, in the natural state, a side face in the second concave portion is inclined by more than 45° with respect to a direction of movement of the elongated member upon insertion and pulling off.
According to the configuration, when the elongated member is inserted into the valve body for the first time after sterilization, the first slit and the second slit exfoliate more readily, and damage to the first slit or the second slid can be prevented with a higher degree of certainty and the elongated member can be inserted into the valve body more readily.

Preferably, in the medical tool and valve body of the present invention, in the natural state, a bottom face in the second concave portion is a flat face substantially perpendicular to a direction of movement of the elongated member upon insertion and pulling off.
According to the configuration, in the state wherein the valve body is mounted on a mounting section, the second concave portion is less likely to be closed, and since the second concave portion is not closed, the area over which blocking occurs when radiation sterilization is carried out can be reduced. Further, when the elongated member is inserted into the valve body for the first time after sterilization, the second concave portion can provide a start of exfoliating of the first slit which is in a sticking state. Consequently, upon insertion, the first slit and the second slit exfoliate readily, and damage to the first slit or the second slid can be prevented. Further, the penetration resistance upon insertion decreases, and the elongated member can be inserted into the valve body readily.

Preferably, in the medical tool and valve body of the present invention, in a state wherein the valve body is mounted on the mounting section, the second concave portion is not closed.
According to the configuration, the area over which blocking occurs when radiation sterilization is carried out can be reduced. Further, when the elongated member is inserted into the valve body for the first time after sterilization, the second concave portion can provide a start of exfoliating of the first slit which is in a sticking state. Consequently, upon insertion, the first slit and the second slit exfoliate readily, and damage to the first slit or the second slid can be prevented. Further, the penetration resistance upon insertion decreases, and the elongated member can be inserted into the valve body readily.

Preferably, in the medical tool and valve body of the present invention, in the natural state, the first slit is open, and
the valve body is deformed, when the valve body is mounted on the mounting section, in such a manner as to be compressed toward the intersecting portion, and the first slit is closed thereby.
According to the configuration, upon insertion and pulling off of the elongated member, a superior sliding performance can be obtained, and a superior sealing performance of the opening and closing section can be obtained.

Preferably, in the medical tool and valve body of the present invention, in the natural state, the first slit and the second slit are open, and
the valve body is deformed, when the valve body is mounted on the mounting section, in such a manner as to be compressed toward the intersecting portion, and the first slit is closed thereby.
According to the configuration, upon insertion and pulling off of the elongated member, a superior sliding performance can be obtained, and a superior sealing performance of the opening and closing section can be obtained.

In an example not according to the invention, in a state wherein the valve body is mounted on the mounting section, at least part of the second slit is open.

According to the configuration, the contact area of the inner faces of the second slit decreases, and consequently, the area over which blocking occurs when radiation sterilization is carried out can be reduced further. Consequently, when the elongated member is inserted into the valve body for the first time after sterilization, the second slit exfoliates more readily, and damage to the second slit can be prevented. Further, the penetration resistance upon insertion decreases further, and the elongated member can be inserted into the valve body readily.

In another example, the first slit has a shape of a straight line as viewed in plan.

According to the configuration, the first slit can be opened and closed readily and with certainty.

In another example, the second slit has a shape of a straight line as viewed in plan, and
the first slit and the second slit intersect crosswise with each other.

According to the configuration, a superior sealing performance of the opening and closing section can be obtained, and the opening and closing section can be opened and closed readily and with certainty.

Furthermore according to the invention, the valve body has a pair of third concave portions provided at the opposite end portions of the first slit on the concave face side.

According to the configuration, the contact area of the inner faces of the first slit decreases further, and consequently, the area over which blocking occurs when radiation sterilization is carried out can be reduced further. Consequently, when the elongated member is inserted into the valve body for the first time after sterilization, the first slit exfoliates more readily, and damage to the first slit or the second slit can be prevented. Further, the penetration resistance upon insertion decreases further, and the elongated member can be inserted readily into the valve body.

Further, since the third concave portions are provided, the resistance (sliding resistance) upon insertion and pulling off of the elongated member decreases, and consequently, a superior sliding performance can be obtained.

Further, since the third concave portions are provided at the opposite end portions of the first slit, even if the third concave portions are made comparatively large, this does not have any influence on the sealing performance of the opening and closing section.

In another example, the third concave portions are provided so as to extend over the inside of the first concave portion and the outside of the first concave portion.

According to the configuration, the sliding performance of the elongated member is further improved, and insertion and pulling off of the elongated member can be carried out more readily.

Preferably, in the valve body of the present invention, in the natural state, the total value of the area of the third concave portions as viewed in side elevation parallel to the first slit is greater than the area of the second concave portion.

According to the configuration, the sliding performance of the elongated member is further improved, and insertion and pulling off of the elongated member can be carried out more readily.

Preferably, in the medical tool and valve body of the present invention, in a state wherein the valve body is mounted on the mounting section, the third concave portions are not closed.
According to the configuration, the area over which blocking occurs when radiation sterilization is carried out can be reduced. Consequently, when the elongated member is inserted into the valve body for the first time after sterilization, the first slit and the second slit exfoliate readily, and damage to the first slit or the second slit can be prevented. Further, the penetration resistance upon insertion decreases, and the elongated member can be inserted readily into the valve body.

In another example not according to the invention, the valve body has at least one pair of projections provided on the concave face side and disposed in an opposing relationship to each other with the first slit interposed therebetween
According to the configuration, when the elongated member is moved downwardly and inserted into the valve body, the valve body is curved such that a portion thereof in the proximity of the intersecting portion is displaced downwardly, and the projections individually contact (point-contact) with the outer circumferential face of the elongated member. Consequently, the contact area of the outer circumferential face of the elongated member and the valve body decreases, and the resistance (sliding resistance) when the elongated member is inserted into the valve body decreases. Consequently, the sliding performance of the elongated member is improved, and the elongated member can be inserted into the valve body readily.

(17) In another example, the projections are positioned in the first concave portion.

According to the configuration, when the elongated member is moved downwardly and inserted into the valve body, the valve body is curved such that a portion thereof in the proximity of the intersecting portion is displaced downwardly, and the projections individually contact (point-contact) with the outer circumferential face of the elongated member. Consequently, the contact area of the outer circumferential face of the elongated member and the valve body decreases, and the resistance (sliding resistance) when the elongated member is inserted into the valve body decreases. Consequently, the sliding performance of the elongated member is improved, and the elongated member can be inserted into the valve body readily.

In another example, the projections are provided radially with respect to the intersecting portion as viewed in plan.

According to the configuration, when the elongated member is moved downwardly and inserted into the valve body, the valve body is curved such that a portion thereof in the proximity of the intersecting portion is displaced downwardly, and the projections individually contact (point-contact) with the outer circumferential face of the elongated member. Consequently, the contact area of the outer circumferential face of the elongated member and the valve body decreases, and the resistance (sliding resistance) when the elongated member is inserted into the valve body decreases. Consequently, the sliding performance of the elongated member is improved, and the elongated member can be inserted into the valve body readily.

As an example, the elongated member is a dilator, a catheter or a guide wire.

According to the configuration, when a dilator, a catheter or a guide wire is inserted into the valve body, it can be guided to a portion of the first slit corresponding to the intersecting portion readily and with certainty, and occurrence of damage to the valve body can be prevented. Further, such a situation that the dilator, catheter or guide wire is crushed or deformed at a distal end portion thereof can be prevented. Further, when the dilator, catheter or guide wire is inserted into the valve body for the first time after sterilization, the insertion can be carried out readily.

In another example, in the natural state, the first slit is open and a first space is formed by an inner face of the first slit while the second slit is open and a second space is formed from an inner face of the second slit, and the first space and the second space are communicated with each other through the intersecting portion.

According to the configuration, when the valve body wherein the first space and the second space are communicated with each other through the intersecting portion in the natural state is mounted on the mounting section, the first slit and the second slit are compressed (pressed) individually in a closing direction by the mounting section. Therefore, the first slit is placed into a closely contacting (closed) state wherein the inner faces thereof are pressed against each other. However, by the elastic force of the valve body itself (restoring force tending to open the first slit), the close contact is placed to such a degree that occurrence of blocking is suppressed (or prevented). Similarly, also the second slit is placed into a closely contacting state as the inner faces thereof are pressed against each other. However, by the elastic force of the valve body itself (restoring force tending to open the second slit), the close contact is placed to such a degree that occurrence of blocking is suppressed (or prevented).

If radiation sterilization is carried out in this state, then occurrence of excessive (excessively firm) blocking between the inner faces of the first slit or between the inner faces of the second slit is prevented with certainty. Consequently, when the elongated member is inserted into the valve body for the first time after sterilization, the inner faces of the first slit (also the inner faces of the second slit) are exfoliated readily. Consequently, the penetration resistance of them is reduced, and an operation when the elongated member is inserted and pulled off can be carried out readily. Further, when the elongated member is inserted and pulled off, damage to the first slit or the second slit can be prevented with certainty.

In another example, in a state wherein the valve body is mounted on the mounting section, the valve body is deformed in such a manner as to be compressed at the intersecting portion such that the first slit and/or the second slit are contacted closely at least at inner peripheral faces thereof in the proximity of the intersecting portion with each other so as to be closed.

According to the configuration, if radiation sterilization is carried out in the state wherein the valve body is mounted on the mounting section, then occurrence of excessive (excessively firm) blocking between the inner faces of the first slit or between the inner faces of the second slit is prevented with certainty. Consequently, when the elongated member is inserted into the valve body for the first time after sterilization, the inner faces of the first slit (also the inner faces of the second slit) are exfoliated readily. Consequently, the penetration resistance of them is reduced, and an operation when the elongated member is inserted and pulled off can be carried out readily. Further, when the elongated member is inserted and pulled off, damage to the first slit or the second slit can be prevented with certainty.

In another example, the first space and the second space have a flattened shape.

According to the configuration, a superior sealing performance is obtained, and the opening and closing section can be opened and closed readily and with certainty.

In another example, the intersecting portion has a shape of a hexahedron in the natural state, and the sum total in length of those eight sides from among the 12 sides forming the hexahedron which are nodal lines formed by intersection of the inner faces of the first slit and the second slit is 1.0 to 6.0 mm.

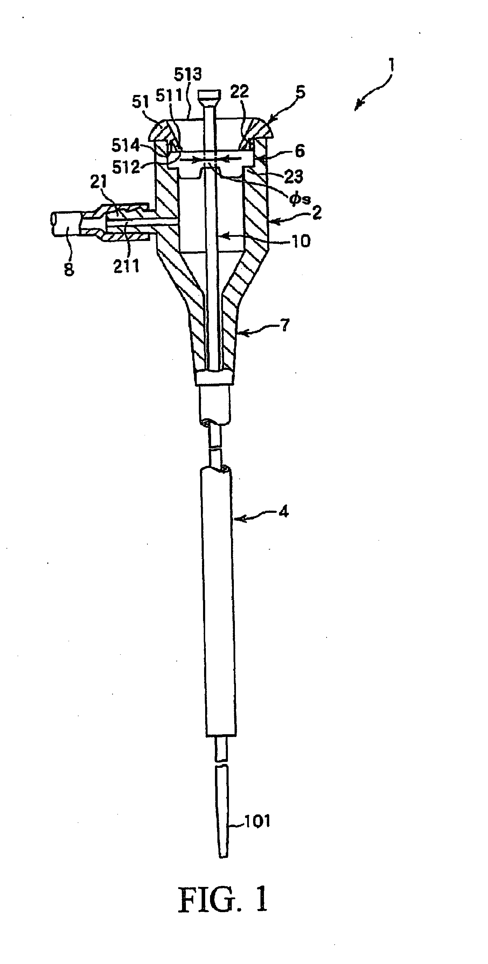

According to the configuration, if the sum total is within such a numeral value range as given above, then the elongated member can be inserted into and pulled off from the valve body suitably (smoothly without causing damage to any of the first slit and the second slit) without depending upon the outer diameter ϕs (refer to FIG. 1) of a portion, which is inserted into a living organism, of the elongated member which is normally used (inserted) in a medical tool. Also, the sealing performance of the opening and closing section in the state wherein the elongated member is fitted in the valve body can be maintained appropriately.

Preferably, in the valve body of the present invention, the intersecting portion is positioned at a central portion of the valve body as viewed in plan.

According to the configuration, when the elongated member is inserted into and pulled off from the valve body, the operation can be carried out stably.

In another example not according to the invention, the first space and/or the second space have a depth which gradually decreases from the intersecting portion toward a direction away from the intersecting portion.

According to the configuration, when the elongated member is inserted into the first slit, the distal end portion of the elongated member is guided so as to be directed to the intersecting portion, and consequently, the insertion operation can be carried out with certainty. Further, the distal end portion of the elongated member is prevented from piercing (penetrating) a portion of the first slit other than the intersecting portion, and further prevented from extending to the second slit. Consequently, damage to the valve body can be prevented.

In another example, the first space and/or the second space have a width which gradually decreases toward the intersecting portion.

According to the configuration, when the elongated member is inserted into the first slit, the distal end portion of the elongated member is guided so as to be directed to the intersecting portion, and consequently, the insertion operation can be carried out with certainty.

In another example, the first slit and the second slit have a shape of a straight line as viewed in plan and intersect crosswise with each other.

According to the configuration, a suitable sealing performance is obtained, and the opening and closing section can be opened and closed readily and with certainty.

Further, in order to attain the object described above, there is provided a medical tool including the valve body of the present invention,
the valve body being installed such that the concave face thereof is exposed to the outer side and the lower face is exposed to the inside of a flow path.
With the medical tool provided by the present invention, when the elongated member is inserted into the valve body, the elongated member can be introduced to a portion of the first slit corresponding to the intersecting portion readily and with certainty, and occurrence of damage to the valve body can be prevented. Further, the distal end portion of the elongated member can be prevented from being crushed or deformed. Further, when the elongated member is inserted into the valve body for the first time after sterilization, insertion of the elongated member can be carried out readily.

### Brief Description of Drawings

[FIG. 1]
   FIG. 1 is a partial vertical sectional view (as regards a valve body, a side elevational view) showing an embodiment where a medical..tool of the present invention is applied to an introduce.
[FIG. 2]

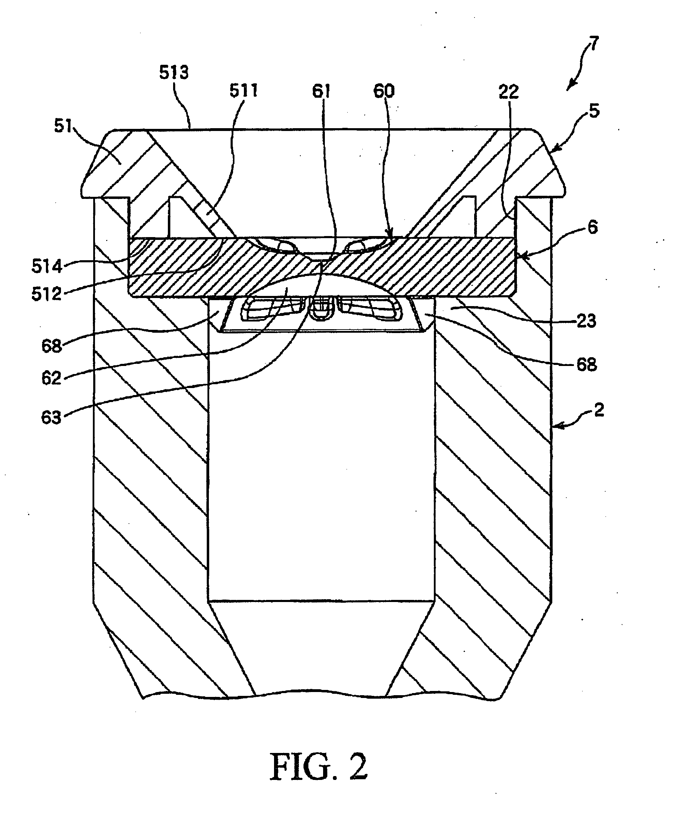

   FIG. 2 is a vertical sectional view showing a hub, a lid member and a valve body of the introducer shown in FIG. 1.
[FIG. 3]

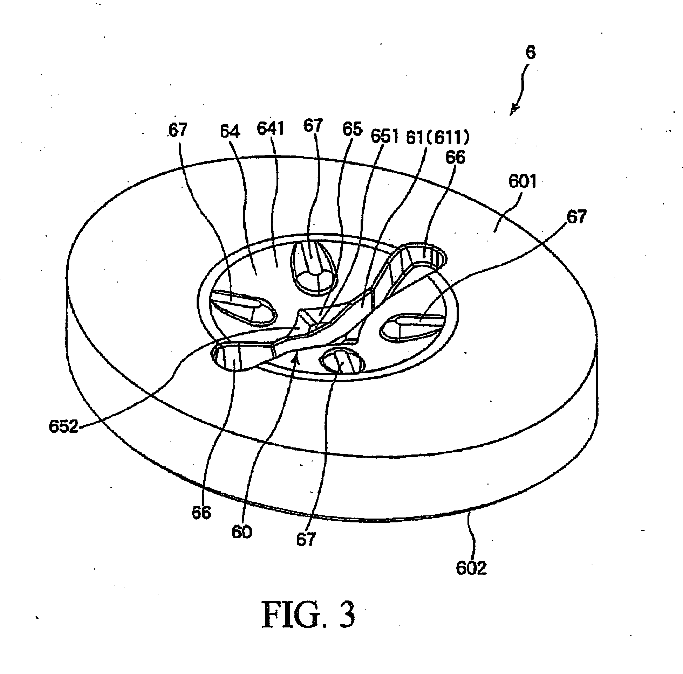

   FIG. 3 is a perspective view of an embodiment of a valve body of the present invention (valve body shown in FIGS. 1 and 2) when the valve body in a natural state is viewed from the upper face side.
[FIG. 4]

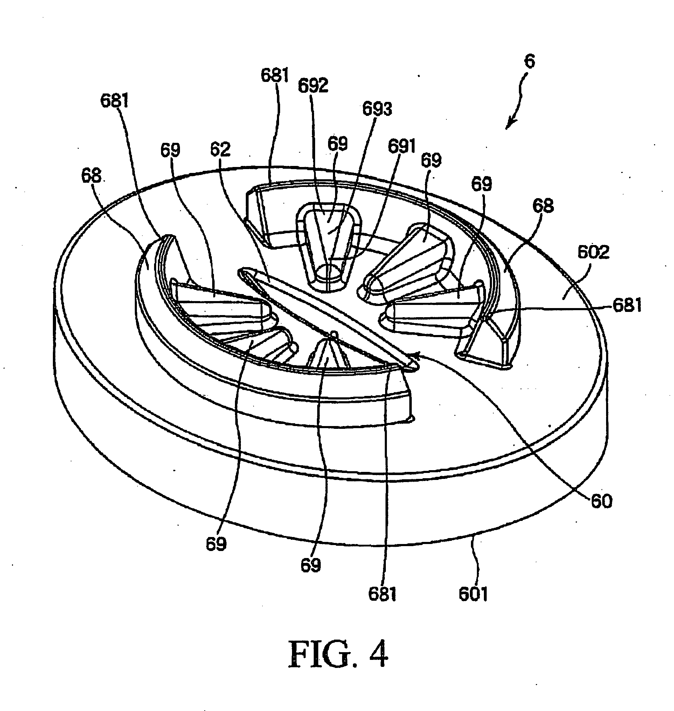

   FIG. 4 is a perspective view of the valve body (in the natural state) shown in FIG. 3 when it is viewed from the lower face side.
[FIG. 5]

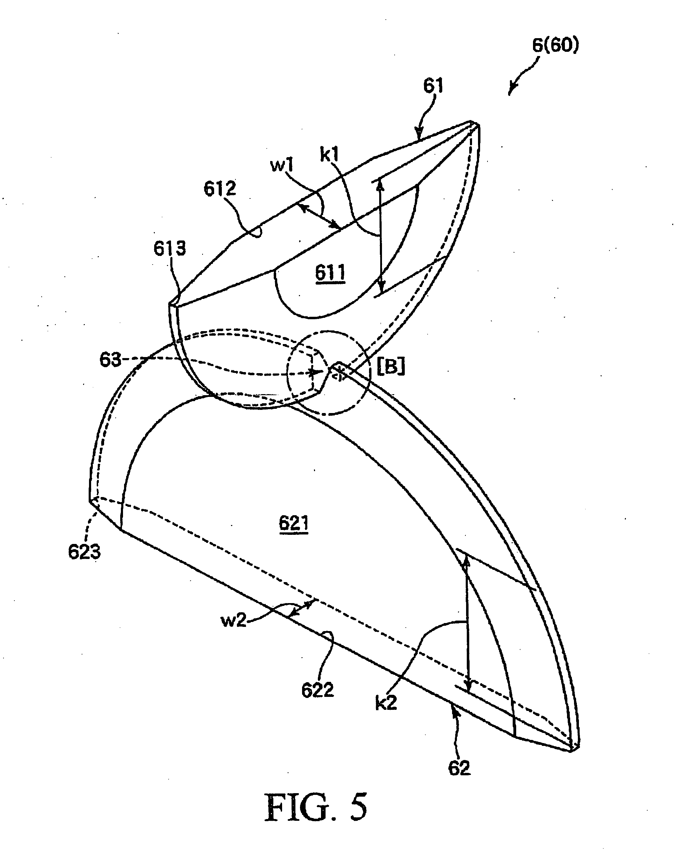

   FIG. 5 is a perspective view showing a first space defined by an inner face of a first slit and a second space defined by an inner face of a second slit of the valve body (in the natural state) shown in FIG. 3.
[FIG. 6]

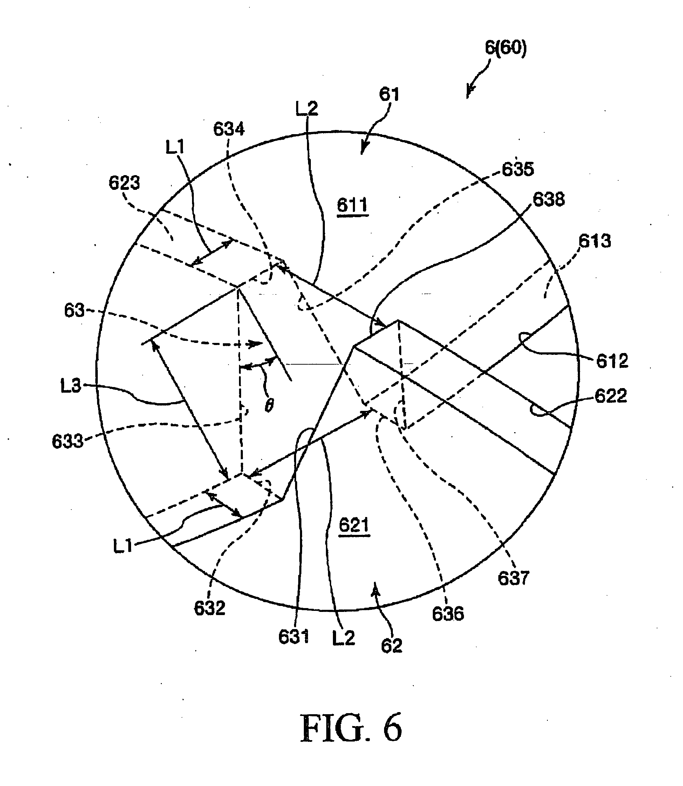

   FIG. 6 is an enlarged detailed view of a region [B] surrounded by an alternate long and short dash line in FIG. 5.
[FIG. 7]

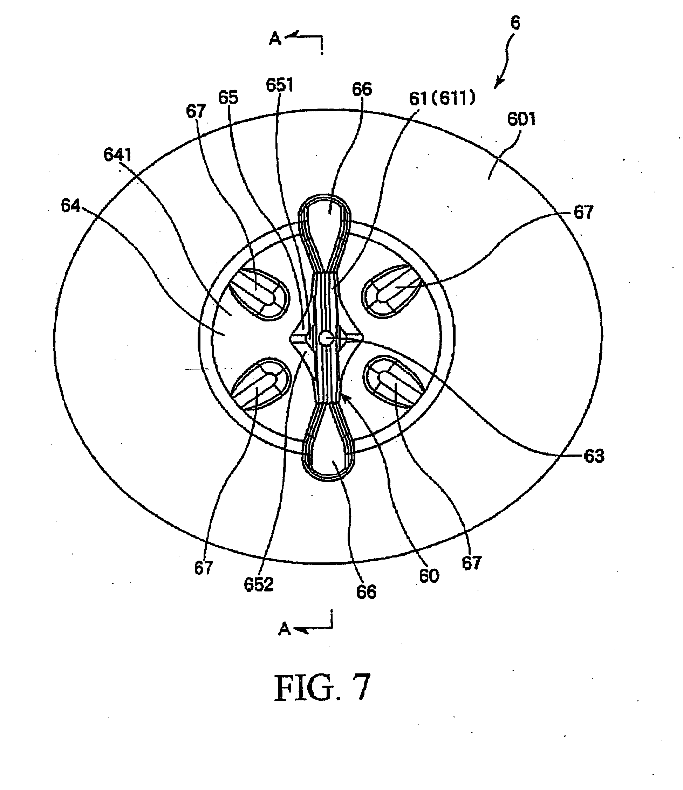

   FIG. 7 is a plan view of the valve body (in the natural state) shown in FIG. 3
[FIG. 8]

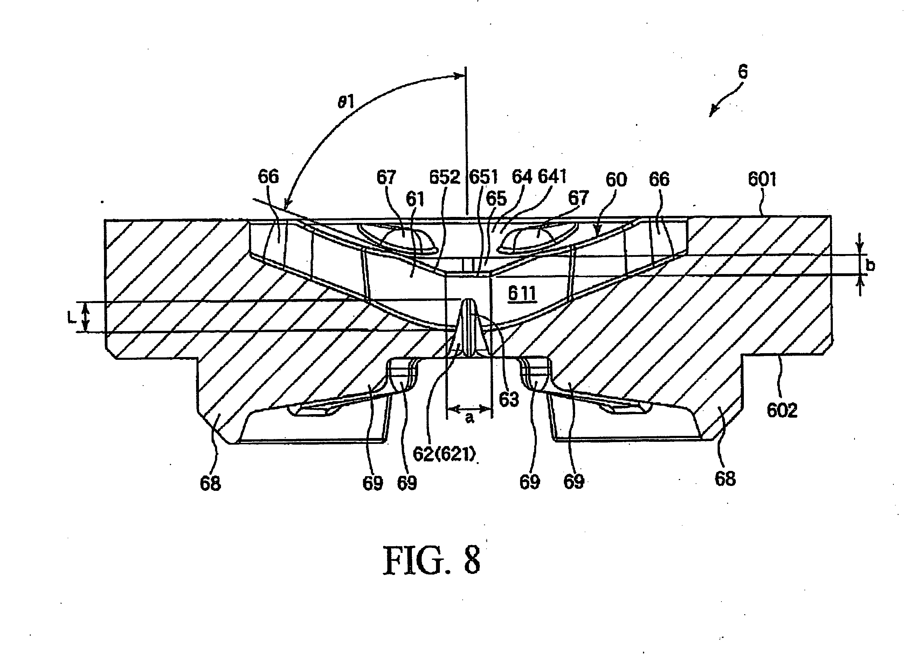

   FIG. 8 is a sectional view taken along line A-A in FIG. 7.
[FIG. 9]

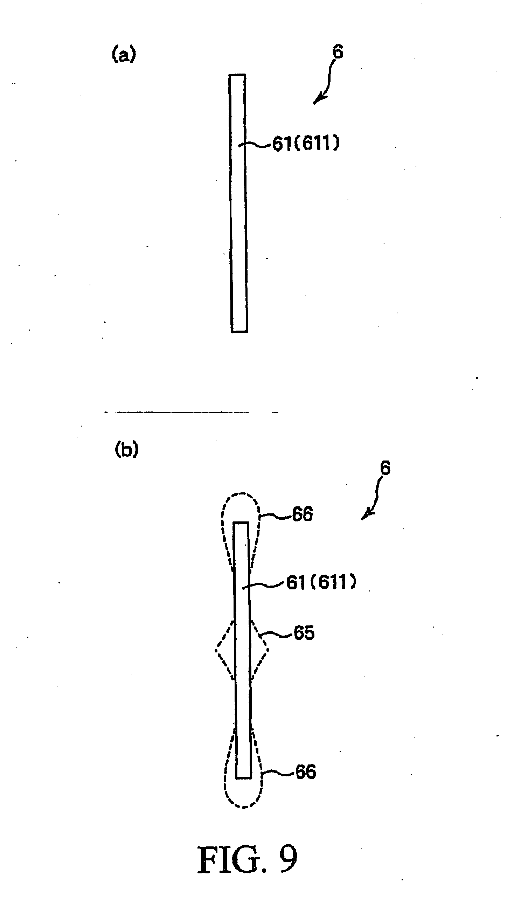

   FIG. 9 is a view which corresponds to a plan view illustrating a second concave portion and a third concave portion of the valve body (in the natural state) shown in FIG. 3.
[FIG. 10]

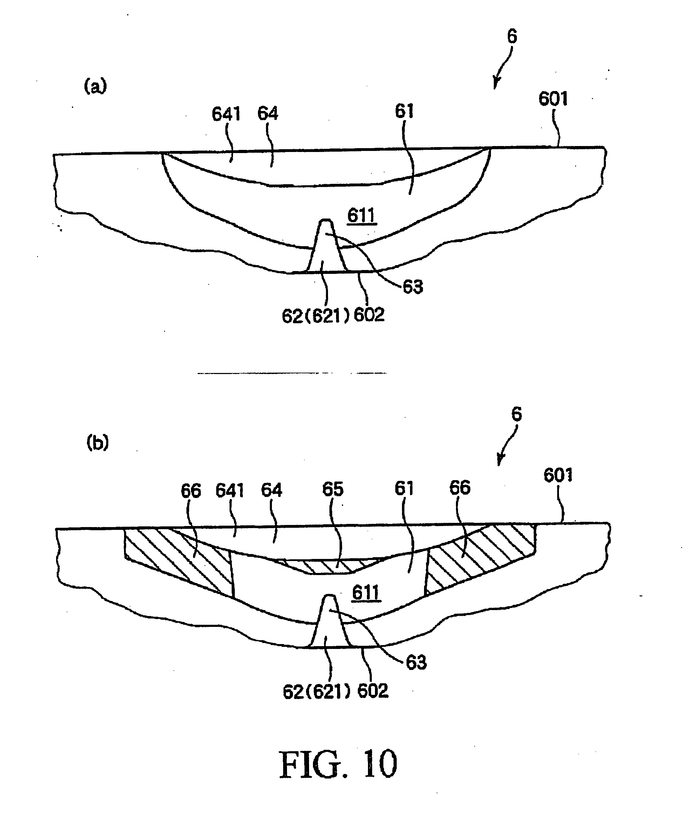

   FIG. 10 is a view which corresponds to a sectional view taken along line A-A in FIG. 7 illustrating the second concave portion and the third concave portion of the valve body (in the natural state) shown in FIG. 3.
[FIG. 11]

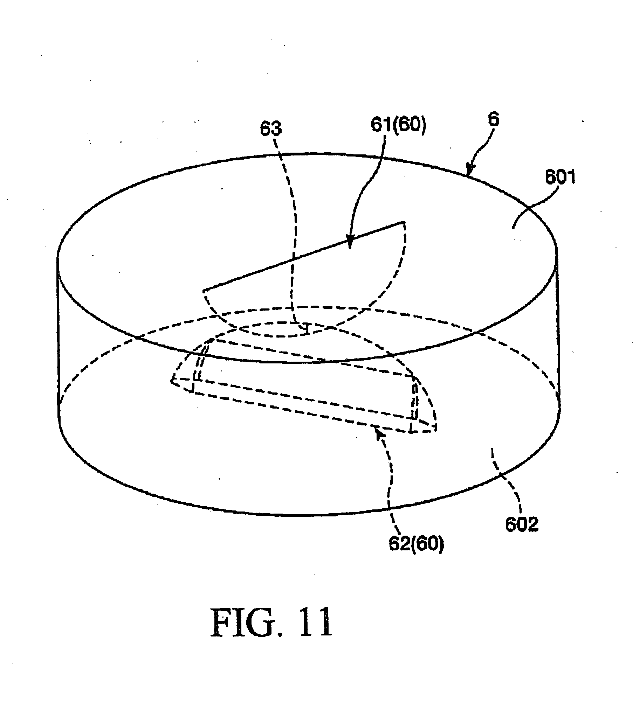

   FIG. 11 is a schematic perspective view of the valve body shown in FIG. 3 but in a mounted state.
[FIG. 12]

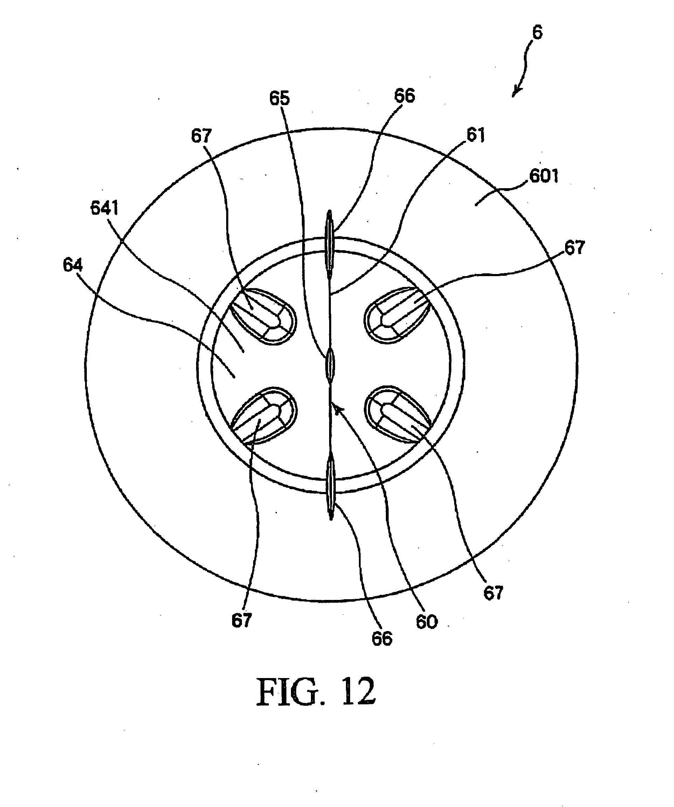

   FIG. 12 is a plan view of the valve body shown in FIG. 3 but in the mounted state.
[FIG. 13]

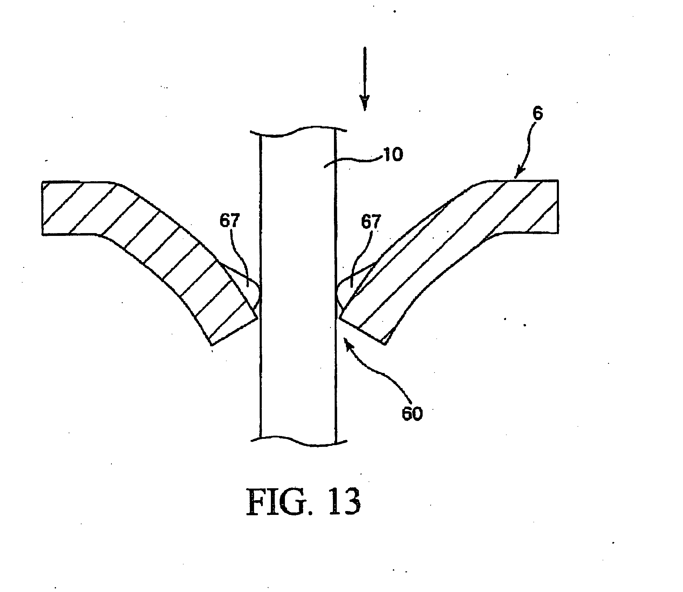

   FIG. 13 is a vertical sectional view schematically illustrating a state when a dilator is fitted into the valve body (in the mounted state) shown in FIG. 12.
[FIG. 14].

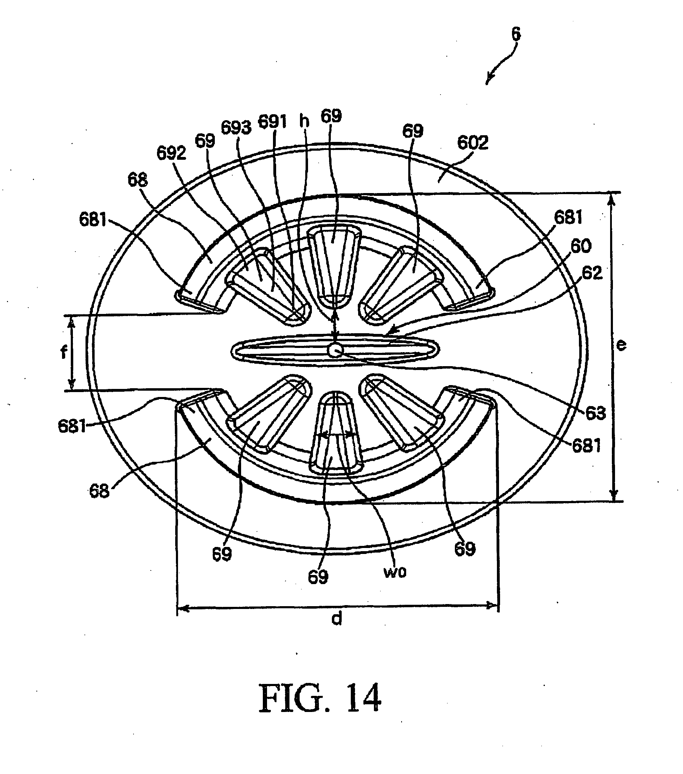

   FIG. 14 is a bottom plan view of the valve body (in the natural state) shown in FIG. 3.
[FIG. 15]

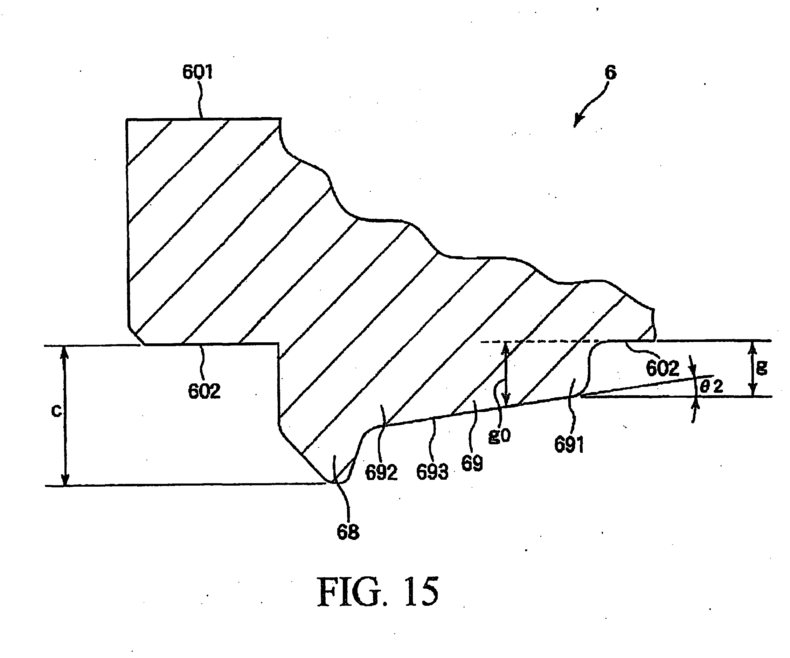

   FIG. 15 is a vertical sectional view showing a rib and a projection on the lower face side of the valve body in FIG. 8.
[FIG. 16]

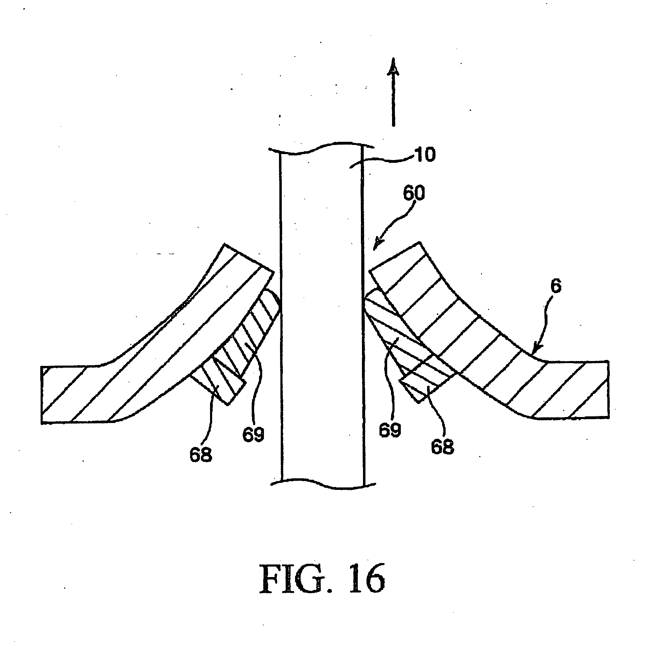

   FIG. 16 is a vertical sectional view schematically illustrating a state when the dilator is pulled off from the valve body (in the mounted state) shown in FIG. 12.

### Best Mode for Carrying Out the Invention

In the following, a valve body and a medical tool of the present invention are described in detail based on a preferred embodiment shown in the accompanying drawings. It is to be noted that, in the present embodiment described below, the medical tool of the present invention is applied representatively to an introducer.

FIG. 1 is a partial vertical sectional view (as regards a valve body, a side elevational view) showing an embodiment where a medical tool of the present invention is applied to an introducer; FIG. 2 is a vertical sectional view showing a hub, a lid member and a valve body of the introducer shown in FIG. 1; FIG. 3 is a perspective view of an embodiment of a valve body of the present invention (valve body shown in FIGS. 1 and 2) when the valve body in a natural state is viewed from the upper face side); FIG. 4 is a perspective view of the valve body (in the natural state) shown in FIG. 3 when it is viewed from the lower face side; FIG. 5 is a perspective view showing a first space defined by an inner face of a first slit and a second space defined by an inner face of a second slit of the valve body (in the natural state) shown in FIG. 3; FIG. 6 is an enlarged detailed view of a region [B] surrounded by an alternate long and short dash line in FIG. 5; FIG. 7 is a plan view of the valve body (in the natural state) shown in FIG. 3; FIG. 8 is a sectional view taken along line A-A in FIG. 7; FIG. 9 is a view which corresponds to a plan view illustrating a second concave portion and a third concave portion of the valve body (in the natural state) shown in FIG. 3; FIG. 10 is a view which corresponds to a sectional view taken along line A-A in FIG. 7 illustrating the second concave portion and the third concave portion of the valve body (in the natural state) shown in FIG. 3; FIG. 11 is a schematic perspective view of the valve body shown in FIG. 3 but in a mounted state; FIG. 12 is a plan view of the valve body shown in FIG. 3 but in the mounted state; FIG. 13 is a vertical sectional view schematically illustrating a state when a dilator is fitted into the valve body (in the mounted state) shown in FIG. 12; FIG. 14 is a bottom plan view of the valve body (in the natural state) shown in FIG. 3; FIG. 15 is a vertical sectional view showing a rib and a projection on the lower face side of the valve body in FIG. 8; and FIG. 16 is a vertical sectional view schematically illustrating a state when the dilator is pulled off from the valve body (in the mounted state) shown in FIG. 12.

It is to be noted that, in the following description, in FIGS. 1 to 3, 5, 6, 8, 10, 11, 13, 15 and 16, the upper side is referred to as "upper" or "proximal end" and the lower side as "lower" or "distal end", and in FIG. 4, the upper side is referred to as "lower" or "distal end" and the lower side as "upper" or "proximal end", for the convenience of description. Further, in FIG. 11, a first slit and a second slit are shown while the other elements are omitted.

The introducer (medical tool) 1 shown in FIG. 1 is used to introduce an elongated member for use in medical care such as, for example, a guide wire or a catheter into a living organism (for example, a blood vessel or the like).

This introducer 1 is composed of a sheath 7 having a hub 2, a sheath tube (tube) 4 in the form of a pipe secured to the distal end side of the hub 2 and sealing means 5, and a dilator (expanding tube) 10 inserted in and used together with the sheath 7 to facilitate introduction of the sheath 7 into a living organism.

The hub 2 is formed from a pipe body.

A side port (connecting portion) 21 is provided on an outer circumferential portion of the hub 2 such that it projects sidewardly. The side port 21 has a form of a pipe, and the hollow (inside) 211 of the side port 21 is communicated with the inside of the hub 2. A tube 8 made of, for example, polyvinyl chloride and having flexibility is connected at one end thereof liquid-tight to the side port 21.

The sheath tube 4 is a member (portion) to be introduced into a living organism and is formed from a thin pipe-like member. This sheath tube 4 is secured to a distal end portion of the hub 2 by fitting or like means. Further, the inside of the sheath tube 4 is communicated with the hollow 211 of the hub 2.

The sealing means 5 for sealing the inside of the hub 2 is provided at a proximal end portion of the hub 2.

The sealing means 5 is composed of a valve body 6 through which the dilator (expanding pipe) 10, a catheter or a guide wire can pass, and an annular (cylindrical) lid member 51 for fixedly supporting the valve body 6 on the hub 2.

The lid member 51 is formed such that it can fit with a proximal end inner circumferential portion 22 of the hub 2, and when the lid member 51 is mounted on the hub 2 and the valve body 6 is fixedly supported on the hub 2., the distal end portion of the lid member 51 is fitted into the proximal end inner circumferential portion 22.

Further, a cylindrical rib 511 is formed on the inner circumferential face portion of the lid member 51 such that it projects in an oblique distal end direction. In particular, the inner diameter and the outer diameter of the rib 511 gradually decrease from the proximal end toward the distal end.

The proximal end portion of the lid member 51 forms an insertion port when an elongated member such as the dilator 10, a catheter or a guide wire is inserted into the sheath 7.

Here, the dilator 10 is described.

The dilator 10 is formed from a pipe body (tube) having flexibility. The dilator 10 is inserted into the sheath 7 and is inserted (introduced) into a living organism in a state wherein it is fixed to the sheath 7. Further, in order to facilitate introduction of the dilator 10, for example, into a blood vessel, the outer diameter of a distal end portion 101 of the dilator 10 reduces (gradually decreases) toward the distal end.

The dilator 10 having such a configuration as described above is removed from the sheath 7 after the sheath tube 4 is inserted into a living organism (blood vessel). Thereafter, a catheter or a guide wire is inserted through an opening (proximal end opening) 513 at the proximal end of the lid member 51.

Incidentally, the sealing means 5 has the valve body 6 as described hereinabove.
As shown in FIGS. 1 to 4 and 8, the valve body 6 is formed from an elastic body which has a pair of end faces and has a form of a plate, in particular, an elastic body which has an upper face (one face) 601 and a lower face (the other face) 602 and has a form of an elliptic or circular film (a form of a disk). This valve body 6 is secured liquid-tight to the hub 2 by being sandwiched by a stepped portion 23 formed at a proximal end portion of the hub 2 and a distal end face 514 of the lid member 51 and a distal end face 512 of the rib 511 (refer to FIGS. 1 and 2). It is considered that, in the introducer 1 (sheath 7), a portion surrounded by the distal end face 514 of the lid member 51 and the distal end face 512 of the rib 511 functions as a "mounting section (installation section)" on which the valve body 6 is to be mounted (installed).

The shape of the valve body 6 in a natural state as viewed in plan, that is, the shape of the valve body 6 before it is mounted on the hub 2 as viewed in plan, exhibits an elliptic shape as shown in FIGS. 7 and 14. Then, the shape of the valve body 6 in a state wherein it is mounted on the hub 2 (the state is hereinafter referred to simply as "mounted state") as viewed in plan, has a circular shape as shown in FIG. 12. In particular, when the.valve body 6 is mounted on the hub 2, it is deformed such that it is compressed toward an intersecting portion 63 hereinafter described along the major axis direction, and consequently, the shape as viewed in plan becomes a circular shape. Here, the "natural state" signifies a state wherein no external force is applied to the valve body 6.

Further, although the dimension of the valve body 6 is not specifically restricted, the major diameter of the valve body 6 in the natural state preferably is approximately 5 to 20 mm and more preferably is approximately 8 to 12 mm. It is to be noted that the major diameter of the valve body 6 in the natural state preferably is greater than the sum total of the inner diameter of the proximal end inner circumferential portion 22 of the hub 2 and the maximum width w1 of a first slit 61. If such.a configuration as just described is adopted, then sealability (sealing performance) of an opening and closing section 60 in the mounted state is obtained.

Further, the minor diameter of the valve body 6 preferably is approximately 4 to 16 mm, and more preferably is approximately 7 to 10 mm.

Further, the thickness of the valve body 6 preferably is approximately 0.5 to 3 mm, and more particularly is approximately 0.8 to 1.5 mm.

Further, the material of the valve body 6 is not restricted specifically other than being elastic, such as, for example, natural rubber, various synthetic rubbers such as isoprene rubber, silicone rubber, urethane rubber, styrene-butadiene rubber, fluororubber and acrylic rubber, and various plastic elastomers of polyamide-based elastomer and polyester-based elastomer may be used.

Further, a first concave portion 64 whose bottom face is a curved concave face (curved concave face) 641 is formed on the upper face.601 side of the valve body 6. In particular, the first concave portion 64 has a bowllike shape. Further, the first concave portion 64 has an elliptic or circular shape as viewed in plan and is disposed such that a central portion thereof and a central portion of the valve body 6 coincide with each other. An end face of the valve body 6 which is on the opposite side to the concave face (bottom face) 641 is the lower face 602, and the valve body 6 is disposed such that the concave face 641 (upper face 601) is exposed (positioned) on the outer side of the sheath 7 and the lower face 602 is exposed in a flow path (in the hub 2) of the sheath 7.

When the dilator (elongated member) 10 is inserted into the valve body 6, the distal end portion 101 of the dilator 10 can be introduced to a portion of the first slit 61 corresponding to the intersecting portion 63 hereinafter described by the first concave portion 64. Consequently, damage to any other portion than the first slit 61 and a second slit 62 by the dilator 10 can be prevented. Further, it is possible to prevent the distal end portion 101 of the dilator 10 from being crushed or deformed.

Further, although the dimension of the first concave portion 64 is not restricted specifically, for example, the depth (maximum depth) of a central portion of the first concave portion 64 preferably is greater than 0.3 mm, more preferably is greater than 0.4 mm, and further preferably is approximately 0.4 to 0.7 mm.

As shown in FIGS. 3 to 8, 11 to 14 and 16, the valve body 6 has an opening and closing section 60 which is opened or closed in response to insertion or pulling off of the dilator 10 (elongated member).

The opening and closing section 60 is composed of a first slit 61, a second slit 62, a second concave portion 65 and two third concave portions 66.

The first slit 61 is formed such that it extends to the concave face 641 (upper face 601) but does not extend to the lower face 602. In other words, the first slit 61 is formed such that it extends only to the concave face 641 (upper face 601) from the inside of the valve body 6.

This first slit 61 is formed as a straight line which coincides with (or extends in parallel to) the minor axis of the valve body 6 as viewed in plan and is included in the first concave portion 64 as viewed in plan. In other words, the first slit 61 is formed in a straight line from one end portion to the other end portion of the first concave portion 64. Consequently, in the mounted state, the first slit 61 (opening and closing section 60) can be opened and closed readily and with certainty.

As shown in FIG. 5 (also in FIGS. 3 and 7), the first slit 61 is opened (open) in the natural state. Consequently, in the first slit 61, a first space 611 is formed from inner faces 612 of the same. This first space 611 has a semicircular disk shape (flattened shape). The first slit 61 is deformed in such a manner that it is compressed toward the intersecting portion 63 along the major axis direction when the valve body 6 is mounted on the hub 2, and consequently is closed thereby. In other words, in the mounted state, the first slit 61 is closed.

Further, the second slit 62 is formed such that it extends to the lower face 602 but does not extend to the concave face 641 (upper face 601). In other words, the second slit 62 is formed such that it extends only to the lower face 602 from the inside of the valve body 6.

This second slit 62 is formed such that it coincides with (or extends in parallel to) the major axis of the valve body 6 as viewed in plan, that is, the shape thereof indicates a straight line as viewed in plan (refer to FIG. 14). Consequently, in the mounted state, the opening and closing section 60 can be opened and closed readily and with certainty.

As shown in FIG. 5 (also in FIGS. 4 and 14), the second slit 62 is opened (open) in the natural state similarly to the first slit 61. Consequently, in the second slit 62, a second space 621 is formed by the inner faces 612. This first space 621 has a form of a semicircular disk (flattened shape). In the mounted state, the second slit 62 is partly closed (partly open).

The first slit 61 and the second slit 62 having such a configuration as described above partly intersect each other in the inside of the valve body 6. The first space 611 and the second space 621 are communicated with each other (refer to, for example, FIG. 5) through the intersecting portion 63 at which the slits intersect with each other (the intersecting portion 63 between the first slit 61 and the second slit 62).

When the valve body 6 wherein the first slit 61 and the second slit 62 are communicated with each other in this manner in the natural state is mounted on the hub 2, the first slit 61 and the second slit 62 are compressed (pressed) in their closing direction by the proximal end inner circumferential portion 22 of the hub 2 (refer to FIG. 11). Therefore, in the first slit 61, the inner faces 612 are pressed against each other and placed into
a closely contacting (closed) state. However, by the elastic force of the valve body 6 itself (restoring force by which the first slit 61 tends to be opened), the degree of the close contact is reduced to such a degree with which occurrence of blocking is suppressed (or prevented). Similarly to this, also in the second slit 62, inner faces 622 are pressed against each other and placed into a closely contacting state. However, by the elastic force of the valve body 6 itself (restoring force by which the second slit 62 tends to be opened), the degree of the close contact is reduced to such a degree that occurrence of blocking is suppressed (or prevented).

If radiation sterilization (for example, electron beam sterilization, γ ray sterilization, X-ray sterilization or the like) is carried out, then occurrence of excessive (excessively firm) blocking is prevented from occurring between the inner faces 612 of the first slit 61 or between the inner faces 622 of the second slit 62 with certainty. It is to be noted that, even with silicone rubber with which a sticking phenomenon of inner faces occurs conspicuously, occurrence of blocking is prevented with certainty. Consequently, when an elongated member such as the dilator 10 is inserted into the valve body 6 for the first time after the sterilization (hereinafter referred to simply as "upon first time insertion"), the inner faces 612 (also the inner faces 622) exfoliate from each other readily, and consequently, the penetration resistance is reduced and an operation when an elongated member is inserted and pulled off can be carried out readily. Further, when an elongated member is inserted and pulled off, damage to the first slit 61 or the second slit 62 can be prevented with certainty.

As shown in FIG. 5, the depth k1 of the first space 611 gradually decreases toward the direction away from the intersecting portion 63. In other words, a potion (bottom face 613) of the first slit 61 which becomes the bottom of the inner faces 612 is curved arcuately. Consequently, when the dilator 10 is inserted into the first slit 61, the distal end portion 101 of the dilator 10 is guided so as to be directed toward the intersecting portion 63, and consequently, the insertion operation can be carried out with certainty. Further, the distal end portion 101 of the dilator 10 is prevented from piercing (penetrating) a portion of the first slit 61 other than the intersecting portion 63 until it comes to the second slit 62, and consequently, damage to the valve body 6 can be prevented.

Further, the width w1 (refer to FIG. 5) of the first space 611 gradually decreases toward the bottom face 613 (intersecting portion 63). Consequently, when the dilator 10 is inserted into the first slit 61, the distal end portion 101 of the dilator 10 is guided so as to be directed to the intersecting portion 63, and consequently, the insertion operation can be carried out with certainty.

Further, the maximum depth k1 of the first slit 61 is not restricted specifically, and preferably is, for example, approximately 35 to 90% of the thickness of the valve body 6 and more preferably is approximately 40 to 80%.

Further, although the bottom face 613 of the first slit 61 in the configuration shown in FIG. 5 is a curved face curved arcuately, the shape of the bottom face 613 is not limited to this, but the bottom face 613 may have, for example, a V shape whose apex (vertex) is the intersecting portion 63.

As shown in FIG. 5, the depth k2 of the second space 621 gradually decreases toward the direction away from the intersecting portion 63 similarly to the first space 611. In other words, a portion (bottom face 623) of the second slit 62 which is the bottom of the inner faces 622 is curved arcuately.

Further, the width w2 (refer to FIG. 5) of the second space 621 gradually decreases toward the bottom face 623 (intersecting portion 63).

The second slit 62 is pressed, in the mounted state, in the direction in which it is closed, that is, in the minor axis direction and is pressed also in its opening direction, that is, in the major axis direction. Therefore, as shown in FIG. 11, in the second slit 62 in the mounted state, a position at which the inner faces 622 closely contact with each other (are closed) and another portion at which the inner faces 622 remain spaced away from each other are formed.

Consequently, the contact area of the inner faces 622 of the second slit 62 decreases, and therefore, the area of a portion at which blocking is likely to occur when radiation sterilization is carried out (area over which the inner faces 622 stick to each other) can be decreased. Consequently, since the inner faces 622 of the second slit 62 exfoliate readily from each other, occurrence of damage to the second slit 62 is prevented, and the dilator 10 can be inserted readily into the valve body 6.

In this manner, from between the first slit 61 and the second slit 62 in the valve body 6, the first slit 61 which is positioned on the insertion side of an elongated member exhibits a higher degree of close contact than the second slit 62. Here, the "degree of close contact" signifies the close contact area and the close contacting force between the inner faces.
It is to be noted that, by adjusting the pressing force (close contacting force) between the inner faces 612 of the first slit 61 in the mounted state, blocking of the portion of the first slit 61 when radiation sterilization is carried out can be suppressed to the minimum while the sealing performance of the opening and closing section 60 is assured.

Further, a portion at which the inner faces 622 are closed is formed in the second slit 62 in the mounted state as described above, and the sealability (sealing performance) of the opening and closing section 60 is obtained by cooperation with the first slit 61 closed in the mounted state. In other words, the liquid-tightness of the inside of the hub 2 is assured, and leakage of liquid (for example, blood) filled in the hub 2 can be prevented with certainty.

The second slit 62 may be entirely closed in the mounted state similarly to the first slit 61.

Further, the maximum depth k2 of the second slit 62 is not restricted specifically and preferably is, for example, approximately 35 to 90% of the thickness of the valve body 6, and more preferably is approximately 40 to 80%.

Further, while the bottom face 623 of the second slit 62 in the configuration shown in FIG. 5 is formed as a curved face curved arcuately, the shape of the bottom face 623 is not limited to this, and for example, the bottom face 623 may have such a V shape whose apex (vertex) is the intersecting portion 63.

As shown in FIG. 5, the first slit 61 and the second slit 62 intersect crosswise with each other at the portion at which the depth of them is in the maximum such that the intersecting angle becomes 90°. It is to be noted that naturally the intersecting angle of these slits 61 and 62 is not limited to 90°.

The intersecting portion 63 between the first slit 61 (first space 611) and the second slit 62 (second space 621) is shared by both of the first slit 61 and the second slit 62 and is set to a middle portion (central portion) of the valve body 6 as viewed in plan. In other words, the intersecting portion 63 is positioned at a central portion of the first concave portion 64 as viewed in plan and is included in the first concave portion 64. Consequently, when the dilator 10 is inserted into and pulled off from the valve body 6 (opening and closing section 60), the operation can be carried out stably.
It is to be noted that, although the length L (refer to FIG. 8) of the intersecting portion 63 is not restricted specifically, it preferably is, for example, approximately 10 to 50% of the thickness of the valve body 6, and more preferably is approximately 15 to 35%. In the following, the intersecting portion 63 is described in detail.

As shown in FIG. 6 (also in FIG. 5), the intersecting portion 63 is a space having a shape of a hexahedron in the natural state. This intersecting portion 63 (hexahedron) has 12 sides which form the same, and the sides include eight sides which are given as nodal lines formed by intersection of the inner faces 612 of the first slit 61 and the inner faces 622 of the second slit 62 (in FIG. 6, the sides 631, 632, 633, 634, 635, 636, 637 and 638).

Although the sum total of the lengths of the sides 631 to 638 is not restricted specifically, it preferably is 1.0 to 6.0 mm and more preferably is 1.5 to 4.5 mm. If the sum total is within such a numeral value range as just mentioned, an elongated member which is normally used (inserted) in the introducer 1 can be inserted into and pulled off from the valve body 6 appropriately (smoothly without damaging the first slit 61 and the second slit 62) without relying upon the outer diameter ϕs (refer to FIG. 1) of a portion of the elongated member to be inserted into a living organism. Further, in a state wherein the elongated member is fitted in the valve body 6, also the sealing performance of the opening and closing section 60 is maintained properly.

Although the length L1 of the sides 632, 634, 636 and 638 which extend in parallel to the upper face 601 and the lower face 602 of the valve body 6 from among the sides 631 to 638 is not restricted specifically, it preferably is 0.1 to 0.5 mm. If the length L1 is within such a numerical value range as just mentioned, then no edge exists at the intersecting portion 63. Accordingly, even if the distal end portion 101 of the dilator 10 strikes the bottom face 613, it is prevented with certainty from making a starting point of damage to the first slit 61 and the second slit 62.

Further, although the length L2 of the four sides other than the nodal lines (distance between the side 634 and the side 638 and distance between the side 632 and
the side 636) is not restricted specifically, it preferably is 0.1 to 0.7 mm.

Further, although the length L3 of the intersecting portion 63 which extends perpendicularly to the upper face 601 and the lower face 602 of the valve body 6 is not restricted specifically, it preferably is 0.2 to 0.6 mm.

Further, although the angle θ defined by the direction of the length L3 and the sides 631, 633, 635 and 637 which do not extend in parallel to the upper face 601 and the lower face 602 of the valve body 6 is not restricted specifically, it preferably is 5 to 40°. Where the angle θ is within such a numeral value range as just mentioned, then when the valve body 6 is mounted on the hub 2, occurrence of excessive blocking.between the inner faces 612 of the first slit 61 or between the inner faces 622 of the second slit 62 is prevented with certainty. Further, the penetration resistance is reduced and an operation when an elongated member is inserted and pulled off can be carried out readily.

It is to be noted that, although the outer diameter ϕs with which fitting of an elongated member into the opening and closing section 60 of the valve body 6 is permitted is not restricted specifically, for example, the maximum value of the same preferably is 6 to 7 mm and more preferably is 6.5 to 7 mm. Meanwhile, the minimum value preferably is 0.1 to 0.3 mm and more preferably is 0.1 to 0.2 mm.

Although the formation method of the first slit 61 and the second slit 62 is not restricted specifically, preferably the valve body 6 is molded, for example, by compression molding, LIM molding, transfer molding or the like, and thereupon, the first slit 61 and the second slit 62 are preferably formed simultaneously.

Further, as described hereinabove, the opening and closing section 60 (valve body 6) has the second concave portion 65 and the third concave portions 66. The second concave portion 65 and the third concave portions 66 are formed on the concave face 641 (upper face 601) side.

Here, in order to facilitate recognition of the second concave portion 65 and the third concave portions 66 (in order that the first slit 61 and the second concave portion 65 and third concave portions 66 can be distinguished readily from each other), only the first slit 61 of the valve body 6 is shown in FIG. 9(a), and the first slit 61, second concave portion 65 and third concave portions 66 of the valve body 6 are shown in FIG. 9(b). Further, in FIGS. 9(a) and 9(b), the first slit 61 is indicated by a solid line and the second concave portion 65 and the third concave portions 66 are indicated by dotted lines (broken lines).

From a similar reason, the valve body 6 on which the second concave portion 65 and the third concave portions 66 are not formed is shown in FIG. 10(a), and the valve body 6 on which the second concave portion 65 and the third concave portions 66 are formed is shown in FIG. 10(b). Further, in FIGS. 10(a) and 10(b) slanting lines, which do not exist on a proper sectional view of .the valve body, are applied only to the second concave portion 65 and the third concave portions 66.

As shown in FIGS. 3 and 7 to 10, the second concave portion 65 is formed at a middle portion of the valve body 6 on the concave face 641 (upper face 601) side, that is, a location (position) of the first concave portion 64 corresponding to the intersecting portion 63. The second concave portion 65 is formed, in the thicknesswise direction of the valve body 6 (in the upward and downward direction), to an intermediate portion of the first slit 61 but does not extend to the second slit 62. Further, at the opposite end portions of the first slit 61 of the valve body 6 on the concave face 641 side, the third concave portions 66 are formed.

The second concave portion 65, third concave portions 66 and first slit 61 are communicated with each other in the natural state and generally form a single groove (concavity).

Further, while, in the natural state, the second concave portion 65 exhibits a diamond shape (quadrangle) (refer to FIGS. 3 and 7), the shape of the second concave portion 65 is not limited to the quadrangular shape, but may be a circular shape. A bottom face 651 in the second concave portion 65 is a flat face substantially perpendicular to the moving direction (longitudinal direction of the dilator 10) upon insertion and pulling off of the dilator 10, and side faces 652 are inclined by a predetermined angle with respect to the moving direction upon insertion and pulling off of the dilator 10 (refer to FIG. 8). The inclination angle θ1 (refer to FIG. 8) of the side faces 652 with respect to the moving direction preferably is 45° or more in the natural state and more preferably is 60° or more, and further preferably is approximately 60 to 80°.

Meanwhile, although the dimension of the second concave portion 65 is not restricted specifically, preferably it is set to such a value that, when the dilator 10 is inserted into the valve body 6, the distal end portion 101 of the dilator 10 does not catch the second concave portion 65, and for example, the length a (refer to FIG. 8) of the first slit 61 of the bottom face 651 preferably is set to a value smaller than the outer diameter of the distal end portion 101 of the dilator 10. Specifically, the length a of the first slit 61 of the bottom face 651 preferably is approximately 0.2 to 0.4 mm in the natural state. Further, the depth b of the second concave portion 65 in the natural state preferably is 0.1 mm or more, and more preferably is approximately 0.1 to 0.2 mm.

As shown in FIG. 12, when the second concave portion 65 is mounted on the hub 2, it is deformed so as to be compressed toward the intersecting portion 63 along the major axis direction, and consequently, it is deformed such that the width thereof decreases. However, in the mounted state, the second concave portion 65 does not fully closely contact with the intersecting portion 63.

By this second concave portion 65, the contact area of the inner faces 612 of the first slit 61 is decreased, and consequently, when radiation sterilization is carried out, the area over which blocking occurs can be reduced. Further, upon insertion for the first time, the second concave portion 65 makes a start of exfoliating at the first slit 61 which is in a sticking state. Consequently, upon insertion for the first time, exfoliating at the first slit 61 occurs readily, and otherwise possible damage to the first slit 61 or the second slit 62 can be prevented. Further, the penetration resistance upon insertion for the first time decreases, and the dilator 10 can be inserted readily into the valve body 6.

Further, in the natural state, the pair of third concave portions 66 have a rather rounded shape as viewed in plan. Further, each of the third concave portions 66 is formed so as to extend over the inside and the outside of the first concave portion 64 (extends from the inside of the first concave portion 64 to the outside of the first concave portion 64).

As shown in FIG. 12, each of the third concave portions 66 is deformed such that, when the valve body 6 is mounted on the hub 2, the third concave portion 66 is compressed toward the intersecting portion 63 along the major axis direction, and consequently, it is deformed such that the width thereof decreases. However, in the mounted state, the third concave portion 66 does not fully closely contact with the intersecting portion 63.

By those third concave portions 66, the contact area of the inner faces 612 of the first slit 61 is decreased, and consequently, the area over which blocking occurs when radiation sterilization is carried out can be decreased. Consequently, upon insertion for the first time, the first slit 61 exfoliates readily, and occurrence of damage to the first slit 61 or the second slit 62 can be prevented. Further, the penetration resistance upon insertion for the first time decreases, and the dilator 10 can be inserted into the valve body 6 readily.

Further, since the third concave portions 66 are provided, the volume of the opposite end portions of the first slit 61 (first space 611) of the valve body 6 decreases, the resistance (sliding resistance) when the dilator 10 is inserted into the valve body 6 or pulled out from the valve body 6 decreases. Consequently, the sliding performance of the dilator 10 is enhanced, and it is possible to insert the dilator 10 into the valve body 6 or pull off the dilator 10 from the valve body 6 readily.

Further, since the third concave portions 66 are provided at the opposite end portions of the first slit 61, even if the third concave portions 66 are made comparatively large, this does not have any influence on the sealing performance of the opening and closing section 60.

Further, as shown in FIG. 8, the total value of the areas of the third concave portions 66 on a vertical section taken along the first slit 61 (as viewed in side elevation parallel to the first slit 61) in the natural state preferably is greater than the area of the second concave portion 65, and more preferably, the area of each of the third concave portions 66 is greater than the area of the second concave portion 65.

Consequently, the sliding performance of the dilator 10 is further improved, and it is possible to insert the dilator 10 into the valve body 6 or pull off the dilator 10 from the valve body 6 more readily.

Further, while, in the configuration shown, the shapes and the dimensions of the pair of third concave portions 66 are set so as to be same as each other, they may be different from each other.

Further, although the dimensions of the third concave portions 66 are not restricted specifically, for example, the depths of the third concave portions 66 in the configuration shown are set equal to the depth of the first slit 61.

Further, on the concave face 641 side of the valve body 6, a plurality of (in the configuration shown, four) projections (second projections) 67 are formed..The projections 67 are positioned in the first concave portion 64 and disposed such that they are opposed to each other through the first slit 61.

In the configuration shown, the four projections 67 are disposed such that they are line-symmetrical with respect to the first slit 61 and are point-symmetrical with respect to the intersecting portion 63 (center of the valve body 6) as viewed in plan. Further, the projections 67 are formed radially from the intersecting portion 63 as viewed in plan.

Further, while, in the configuration shown, the shapes and the dimensions of the projections 67 are set so as to be same as each other, they may be different from each other.

Further, although the dimensions of the projections 67 are not restricted specifically, for example, the height preferably is approximately 0.1 to 0.5 mm and more preferably is approximately 0.2 to 0.3 mm.

It is to be noted that, while, in the configuration shown, two pairs of projections 67 are provided, the number of the projections 67 is not restricted to this, but only one pair of projections 67 may be provided or three or more pairs of projections 67 may be provided. Further, the number of projections 67 may be an odd number without being disposed in symmetrical arrangement with respect to the first slit 61.

As shown in FIG. 13, when the dilator 10 is moved downwardly and inserted into the valve body 6, the valve body 6 is curved such that a central portion thereof is displaced downwardly and the projections 67 contact (point-contact) with the outer circumferential face of the dilator 10. Consequently, the contact area between the outer circumferential face of the dilator 10 and the valve body 6 decreases, and the resistance (sliding resistance) when the dilator 10 is inserted into the valve body 6 decreases. Consequently, the sliding performance of the dilator 10 is improved, and the dilator 10 can be inserted into the valve body 6 readily.

Further, when the dilator 10 is inserted into the valve body 6, the dilator 10 is guided by the projections 67 such that it is directed to a central portion of the valve body 6, that is, toward the intersecting portion 63. Consequently, the dilator 10 can be inserted into the valve body 6 readily, and damage to the valve body 6 can be prevented.

Further, as shown in FIGS. 4, 8 and 14 to 16, the valve body 6 has ribs (wall portions) 68 and projections (first projections) 69 formed on the lower face 602 side thereof. The sliding performance of the dilator 10 is improved by the ribs 68 and the projections 69, and insertion and pulling off of the dilator 10 can be carried out readily and the sealability (sealing performance) of the opening and closing section 60 is improved. In the following, the ribs 68 and the projections 69 are described.

A pair of ribs 68. extending along the lower face 602 are formed on the lower face 602 side of the valve body 6. The ribs 68 have an arcuate shape. Further, the ribs 68 are disposed in an opposing relationship to each other with the second slit 62 interposed therebetween,
and in the mounted state, the ribs 68 contact at the outer peripheral faces (outer sides) thereof with an inner circumferential face of the hub 2.

In the configuration shown in FIG. 14, the ribs 68 are disposed such that they are line-symmetrical with each other with respect to the second slit 62 and point-symmetrical with each other with respect to the intersecting portion 63 as viewed in plan.

As shown in FIG. 2, when the valve body 6 is mounted on the hub 2, the ribs 68 contact at the outer peripheral faces thereof with the inner circumferential face of the hub 2. Consequently, when the valve body 6 is deformed such that it is compressed toward the intersecting portion 63 along the major axis direction, the second slit 62 is prevented from being opened further. Consequently, the sealing performance of the opening and closing section 60 is maintained.

Further, when the inside of the hub 2 is placed into a negative pressure state, although the valve body 6 is pulled (downwardly) into the hub.2, since the ribs 68 contact at the outer peripheral face thereof with the inner circumferential face of the hub 2 and the valve body 6 is reinforced by the ribs 68, deformation of the valve body 6 is restrained and the second slit 62 can be prevented from being opened further. Consequently, the sealing performance of the opening and closing section 60 is maintained.

Although the dimensions of the ribs 68 are not restricted specifically, for example, the height c (refer to FIG. 15) of the ribs 68 preferably is 0.3 mm or more and more preferably is approximately 0.3 to 1.3 mm, and further preferably is approximately 0.5 to 1 mm.

Further, the length d (refer to FIG. 14) of the ribs 68 in the direction of the second slit 62 preferably is greater than the length of the second slit 62, and preferably is equal to, for example, approximately 3 to 7 times the length of the second slit 62 and more preferably is equal to approximately 3.5 to 6 times. In the configuration shown, the length d of the ribs 68 is set greater than the length of the second slit 62.

Further, a predetermined gap is provided between end portions 681 of one of the ribs 68 and end portions 681 of the other one of the ribs 68. In other words, the ribs 68 are not formed on an extension line of the second slit 62 as viewed in plan.

Consequently, where the valve body 6 is deformed such that it is compressed toward the intersecting portion 63 along the major axis direction when it is mounted on the hub 2, a relief corresponding to the deformed portion is assured. Consequently, the valve body contacts (closely contacts) uniformly with the hub 2, and the liquid-tightness of the inside of the hub 2 is maintained with certainty.

Further, the resistance (sliding resistance) when the dilator 10 is moved (inserted and pulled off) with respect to the valve body 6 can be prevented from increasing unnecessarily. Consequently, the sliding performance of the dilator 10 is improved, and insertion and pulling off of the dilator 10 can be carried out readily.

The gap distance (refer to FIG. 14) between the end portions 681 of one of the ribs 68 and the end portions 681 of the other of the ribs 68 preferably is such a distance that, in the mounted state, an end portion of one of the ribs 68 and an end portion of the other one of the ribs 68 do not contact with each other. In particular, in the natural state, the gap distance f preferably is approximately 0.5 to 3 mm and more preferably is approximately 1 to 2 mm.

Further, in the natural state, the length e (refer to FIG. 14) between the outer peripheral face of one of the ribs 68 and the outer peripheral phase of the other of the ribs 68 (the twice value of a radius of curvature of the outer peripheral face of the ribs 68) is set a little greater than the inner diameter of the hub 2 at a position at which the hub 2 corresponds to the ribs 68. Consequently, in the mounted state, the portions of the ribs 68 which have the length e are compressed toward the intersecting portion 63 (in a direction in which the second slit 62 is closed) by the inner circumferential face of the hub 2. Consequently, the second slit 62 is closed with certainty.

It is to be noted that the shape of the ribs 68 is not limited to the arcuate shape but may be a curved shape having portions having different curvatures such as an elliptic arc, a straight shape (bar shape) or the like.

Further, while, in the configuration shown, a pair of ribs 68 are provided, the number of ribs 68 is not limited to this, but may be two pairs or more or may be an odd number without being symmetrically disposed with respect to the second slit 62.

Further, as shown in FIGS. 4 and 14, a plurality of (in the configuration shown, six) projections 69 are formed on the lower face 602 side of the valve body 6.
The projections 69 are disposed between the pair of ribs 68 and the second slit 62. In other words, the projections 69 are positioned on the inner side of the ribs 68 (between the pair of ribs 68) and are disposed in an opposing relationship to each other with the second slit 62 interposed therebetween.

In the configuration shown in FIG. 14, the six projections 69 are disposed line-sequentially with respect to the second slit 62 and point-symmetrically with respect to the intersecting portion 63 (center of the valve body 6). Further, the projections 69 are formed radially with respect to the center of the intersecting portion 63 as seen in plan and extend from the ribs 68 toward the intersecting portion 63.

Further, the projections 69 are formed at intervals of an equal angle (intervals of 45°). In particular, the pair of projections 69 are formed along the first slit 61 as viewed in plan, and the projections 69 are formed at intervals of an equal angle (intervals of 45°) on the opposite sides of the pair of the projections 69.

As shown in FIG. 16, when the dilator 10 is moved upwardly and pulled off from the valve body 6, the valve body 6 is deformed such that a central portion thereof is displaced upwardly and the projections 69 contact (point-contact) with the outer peripheral face of the dilator 10. Consequently, the contact area of the outer peripheral face of the dilator 10 and the valve body 6 decreases, and the resistance (sliding resistance) when the dilator 10 is pulled off from the valve body 6 decreases. Consequently, the sliding performance of the dilator 10 is improved, and the dilator 10 can be removed from the valve body 6 readily.

Further, particularly when the dilator 10 is inserted in the valve body 6, the valve body 6 is reinforced by the projections 69, and consequently, the second slit 62 become less likely to be opened. The sealing performance of the opening and closing section 60 is improved by this.

Although the shapes and the dimensions of the projections 69 are not restricted specifically, in the configuration shown, distal end portions 691 (end portions on the second slit 62 (intersecting portion 63) side) of the projections 69 are rather rounded, and the projections 69 have a same shape and have a same dimension. In the following description, one projection 69 is described as a representative.

The height g of the distal end portion 691 of the projection 69 preferably is greater than 0.2 mm and more preferably is approximately 0.3 to 0.5 mm.

Where the height g is set greater than the lower limit value mentioned above, not only when an elongated member (dilator 10) having a comparatively great outer diameter is to be pulled off but also when an elongated member having a comparatively small outer diameter is to be pulled off, the projections 69 become liable to contact with the outer peripheral face of the dilator 10.

On the other hand, where the height g is set lower than the upper limit value given above, the increasing amount of the volume of the valve body 6 can be restricted to a comparatively low value. Consequently, the resistance (sliding resistance) when the dilator 10 is moved (inserted and pulled off) with respect to the valve body 6 can be made comparatively low.

Further, the height of a proximal end portion 692 of the projection 69 (end portion on the rib 68 side) is lower than the height c of the rib 68. Consequently, the increasing amount of the volume of the valve body 6 can be set to a comparatively low value, and the resistance (sliding resistance) when the dilator 10 is moved (inserted and pulled off) with respect to the valve body 6 can be made comparatively low.

Further, in the configuration shown, the height (g0 in FIG. 15) of the projection 69 gradually decreases from the outer circumferential portion toward the central portion of the valve body 6, that is, from the rib 68 side toward the second slit 62 (intersecting portion 63) side. Consequently, when the dilator 10 is inserted into the valve body 6, the sliding resistance of the same can be made comparatively low. It is to be noted that the height of the projection 69 (g0 in FIG. 15) may otherwise be fixed.

The inclination angle θ2 of a lower face 693 of the projection 69 with respect to the lower face 602 preferably is approximately 0 to 10° and more preferably is approximately 0 to 8°.

Further, the width (w0 in FIG. 14) of the projection 69 gradually decreases from the rib 68 side toward the second slit 62 side. Consequently, when the dilator 10 is inserted into the valve body 6, increase of the sliding resistance of the same can be suppressed.

Further, the projection 69 does not extend to the second slit 62. Consequently, when the dilator 10 is inserted into the valve body 6, the valve body 6 is liable to be deformed such that a central portion thereof is displaced downwardly to a suitable degree, and the projections 67 of the upper face 601 side and the outer peripheral face of the dilator 10 can be made contact with each other with certainty.

- Further, all of the gap distances h (refer to FIG. 14) between the distal end portions 691 of the projections 69 and the lower face 693 are equal to each other. Consequently, when the dilator 10 is pulled off from the valve body 6, the projections 69 and the outer peripheral face of the dilator 10 can be made contact each other uniformly. As a result, the dilator 10 can be removed from the valve body 6 readily and stably.

Although the gap distance h mentioned above is not restricted specifically, in the natural state, it preferably is approximately 0 to 1.5 mm and more particularly is approximately 0.3 to 0.5 mm.

Further, the projections 69 are not formed on an extension line of the second slit 62 as viewed in plan. Consequently, the resistance (sliding resistance) when the dilator 10 is moved (inserted and pulled off) with respect to the valve body 6-can be prevented from increasing unnecessarily. As a result, the sliding property of the dilator 10 is improved, and insertion and pulling off of the dilator 10 can be carried out readily.
Further, the projections 69 and the ribs 68 are connected to each other. Consequently, deformation of the valve body 6 is suppressed, and the sealability of the opening and closing section 60 is improved. It is to be noted that the projections 69 may otherwise be spaced from the ribs 68.

Further, while, in the configuration shown, three pairs of projections 69 are provided, the number of projections 69 is not limited to this, but one pair, two pairs or four or more pairs may be provided, or the number of projections 69 may be an odd number without being disposed in symmetrical arrangement with respect to the second slit 62. As another preferred configuration of the configuration shown, for example, a modification to the configuration shown wherein the two projections 69 at the central location are not formed but the four projections 69 at the opposite end portions are formed or another modification wherein the four projections 69 at the opposite end portions are not formed but the two projections 69 at the central location.are formed may be used.

Further, although the valve body 6 preferably is formed as a single member from the same material, the configuration of the valve body 6 is not limited to this. For example, two or more different materials may be used to produce the valve body 6 or a plurality of members may be integrated by fusion, adhesion or the like to produce the valve body 6.

As described above, according to the present introducer 1 (valve body 6), insertion and pulling off of the dilator 10 can be carried out readily.

Further, even if insertion and pulling off of the dilator 10 are repeated frequently, occurrence of damage to the first slit 61 or the second slit 62 can be prevented (durability is high), and the sealability (sealing performance) of the opening and closing section 60 is maintained. In other words, the liquid-tightness of the inside of the hub 2 is maintained, and leakage of liquid (for example, blood) filled in the hub 2 can be prevented with certainty.

Particularly, when the dilator 10 is inserted into the valve body 6, the dilator 10 can be introduced to a portion of the first slit 61 corresponding to the intersecting portion 63 readily and with certainty by the first concave portion 64. Consequently, such a situation that any other portion than the first slit 61 and the second slit 62 is pierced by the dilator 10 and a tear (crack) appears with the first slit 61 or the second slit 62 can be prevented, and it is possible to prevent the distal end portion 101 of the dilator 10 from being crushed or damaged.

Further, since the second slit 62 is partly open in the mounted state and the second concave portion 65 and the third concave portions 66 are provided, the area over which blocking occurs when radiation sterilization is carried out can be reduced. Further, when the dilator 10 is inserted into the valve body 6 for the first time after sterilization (upon insertion for the first time), the second concave portion 65 provides a start of exfoliating of the first slit 61 which is in a sticking state. Consequently, upon insertion of the dilator 10, the first slit 61 (opening and closing section 60) exfoliates readily, and the penetration resistance upon insertion decreases and the dilator 10 can be inserted into the valve body 6 readily.

Further, where the third concave portions 66 are provided, since the volume of the opposite end portions of the first slit 61 of the valve body 6 decreases, the resistance (sliding resistance) when the dilator 10 is inserted into the valve body 6 or when the dilator 10 is pulled off from the valve body 6 decreases. Consequently, the sliding performance of the dilator 10 is improved, and the dilator 10 can be inserted into the valve body 6 or pulled off from the valve body 6 readily.

Further, when the dilator 10 is moved downwardly and inserted into the valve body 6, the valve body 6 is curved such that a central portion thereof is displaced downwardly and the projections 67 contact (point-contact) with the outer peripheral face of the dilator 10. Consequently, the contact area of the outer peripheral face of the dilator 10 and the valve body 6 decreases, and the resistance (sliding resistance) when the dilator 10 is inserted into the valve body 6 decreases. Consequently, the sliding performance of the dilator 10 is improved, and the dilator 10 can be inserted into the valve body 6 readily.

Further, when the dilator 10 is inserted into the valve body 6, the dilator 10 is guided by the projections 67 such that it is directed to a central portion of the valve body 6, that is, toward the intersecting portion 63. Consequently, the dilator 10 can be inserted into the valve body 6 readily, and damage to the valve body 6 can be prevented.

Further, the elongated member to be inserted into the sheath 7 through the valve body 6 is not restricted to the dilator 10, but may be, for example, a catheter or a guide wire.

The element to be inserted into and pulled off from the valve body is not limited to a hollow element such as a pipe body but may be, for example, a solid element.
Further, the first slit and the second slit are not limited to those which are open in the natural state, but may be, for example, those which are closed in the natural state.

Further, the first slit and the second slit are not limited to those which have a shape of a straight line as viewed in plan, but only one of the first slit and the second slit may have a shape of a straight line, or the first slit and the second slit may have shapes other than a shape of a straight line.

Further, the medical tool of the present invention is not limited to an introducer..
Further, while both of the first space and the second space are formed such that the depth thereof gradually decreases toward the direction away from the intersecting portion, the configuration of the first space and the second space is not limited to this, and only one space may be formed such that the depth thereof gradually decreases toward the direction away from the intersecting portion.

Further, although the depth described above gradually decreases toward the direction away from the intersecting portion, the depth is not limited to this, but it may be, for example, uniform

Further, although both of the first space and the second space have a portion whose width gradually decreases toward the intersecting portion, the configuration of the first space and the second space is not limited to this, but only one of the spaces may have a portion whose width gradually decreases toward the intersecting portion.

### Working Examples

Now, particular working examples of the valve body of the present invention are described.

### 1. Production of the Sheath (Introducer)

### (Working Example 1)

The valve body shown in FIGS. 3 to 12 was produced by transfer molding using silicone rubber without forming the ribs 68 and the projections 69 of the lower face 602 side, and this valve body was mounted to produce the sheath (introducer) shown in FIGS. 1 and 2. To the first slit and the second slit of the valve body, silicone oil (viscosity: 1,000 cSt) was applied.

Further, the dimensions of individual portions of the valve body in the natural state are such as given below:
Major axis of the valve body: 8.6 mm
Minor axis of the valve body: 7.7 mm
Thickness of the valve body: 1.3 mm
Depth (maximum depth) of the central portion of the first concave portion 64: 0.4 mm
Length a of the bottom face 651 of the second concave portion 65: 0.56 mm
Depth b of the second concave portion 65: 0.1 mm
Inclination angle θ1 of the side face 652 of the second concave portion 65: 65 to 75°

### (Working Example 2)

The working example 2 was similar to the working example 1 except that the third concave portions 66 and the projections 67 were not formed.

### (Working Example 3)

The working example 3 was similar to the working example 1 except that the third concave portions 66 were not formed.

### (Working Example 4)

The working example 4 was similar to the working example 1 except that the projections 67 were not formed.

### (Working Example 5)

The working example 5 was similar to the working example 1 except that the second concave portion 65 was formed in a conical shape.

### (Comparative Example 1)

The comparative example 1 was similar to the working example 1 except that the second concave portion 65 was not formed.

### (Comparative Example 2)

The comparative example 2 was similar to the working example 1 except that the valve body had a circular shape having a diameter of 8 mm as viewed in plan and the first slit and the second slit which were closed in the natural state were formed by cutter working while the first concave portion 65, second concave portion 65, third concave portions 66 and projections 67 were not formed.

### 2. Evaluation

The following evaluation was conducted for the working examples and the comparative examples.

### [2. 1] Evaluation of the First Time Penetration Performance

First, electron beam sterilization (radiation dose: 55 kGy) was carried out for the sheath. Then, the evaluation of the following (1) and (2) was carried out.

(1) A dilator (made by Terumo Corporation) having an outer diameter of 2 mm was inserted into the valve body of the sheath after the electron beam sterilization, and damage to the valve body (a tear at the slit portions) and crush (break) at the distal end portion of the dilator were confirmed using an optical microscope.

Further, in order to evaluate the damage to the valve body, the sealability was confirmed in the following manner in a state wherein a dilator (made by Terumo Corporation) having an outer diameter of 2 mm was inserted in the valve body after the confirmation by visual observation, another state wherein three guide wires having different dimensions were inserted and a further state wherein nothing was inserted. It is to be noted that the three guide wires had outer diameters of 0.89 mm, 0.46 mm and 0.36 mm.

The opening at the distal end portion of the sheath was sealed, and air was injected into the sheath by 0.3 kgf/cm² through the side port and a flow of the air was confirmed in water by visual observation.

For each of the working examples and the comparative examples, the evaluation was carried out with regard to 30 samples with the following criteria.

○: excellent
Δ: good
×: rather bad

(2) For the sheaths after the electron ray sterilization, a dilator (made by Terumo Corporation) having an outer diameter of 2 mm was inserted (penetrated) into the valve body, and the penetration resistance then was measured.

In this instance, a maximum load when the Autograph (produced by Shimadzu Corporation, AG-IS) was used and operated at a cross head speed of 200 mm/min was determined.

For each of the working examples and the comparative examples, the determination was carried out with regard to five samples, and an average value was determined.
It is to be noted that the penetration resistance was evaluated as being good if it is lower than 200 gf.

### [2. 2] Sliding Performance (Sliding Resistance)

A dilator (produced by Terumo Corporation) having an outer diameter of 2 mm was inserted into and pulled off from the sheath after the electron beam sterilization. Thereafter, the sliding resistance was measured when a catheter (produced by Terumo Corporation, Heartcath) having an outer diameter of 1.7 mm was inserted and when the catheter was pulled off.

In this instance, a maximum load when the Autograph (produced by Shimadzu Corporation, AG-IS) was used and operated at a cross head speed of 100 mm/min by 100 mm was determined.

For each of the working-examples and the comparative examples, the determination was carried out with regard to five samples, and an average value was determined.

It is to be noted that the sliding resistance upon insertion was evaluated such that, if it was lower than 150 gf, then the sliding performance was good, and the sliding resistance upon pulling off was evaluated such that, if it was lower than 200 gf, then the sliding performance was good.

### [2. 3] Durability

A catheter (produced by Terumo Corporation, Heartcath) having an outer diameter of 2 mm was inserted into the sheath after electron beam sterilization, and then an operation cycle of back and forth movement of a stroke of 200 mm was repeated by 500 times.

Then, after the catheter was removed from the valve body, the sealing performance was confirmed in the following manner with regard to a state wherein a dilator (produced by Terumo Corporation) having an outer diameter of 2 mm was inserted, another state wherein each of three guide wires having different dimensions was inserted and a further state wherein nothing was inserted. It is to be noted that the three guide wires had outer diameters of 0.89 mm, 0.46 mm and 0.36 mm.

The opening at the distal end portion of the sheath was sealed, and air was injected into the sheath by 0.3 kgf/cm² through the side port and a flow of the air was confirmed in water by visual observation.

For each of the working examples and the comparative examples, the evaluation was carried out with regard to 30 samples with the following criteria.

○: excellent
Δ: good
×: rather bad

The evaluation above mentioned is shown in Table 1.

[Table 1]

**Table 1**

| | Slits | Valve body upper face specification | | | | | Evaluation | | | | | |
|---|---|---|---|---|---|---|---|---|---|---|---|---|
| | | First concave portion | Second concave portion | | Third concave portion | Projection | Penetration performance | | | Sliding performance | | Durability |
| | | | | | | | Penetration resist ance [gf] | Valve body state | Dilat or state | Upon insertion [gf] | Upon pulling off [gf] | |
| Working example 1 | Transfer molding | Yes | Yes | Quadrangular pyramid | Yes | Yes | 150 | ○ | ○ | 135 | 180 | Δ |
| Working example 2 | Transfer molding | Yes | Yes | Quadrangular pyramid | No | No | 150 | Δ | Δ | 150 | 190 | Δ |
| Working example 3 | Transfer molding | Yes | Yes | Quadrangular pyramid | No | Yes | 150 | Δ | Δ | 140 | 190 | Δ |
| Working example 4 | Transfer molding | Yes | Yes | Quadrangular pyramid | Yes | No | 150 | Δ | Δ | 135 | 180 | Δ |
| Working example 5 | Transfer molding | Yes | Yes | Conical shape | Yes | Yes | 180 | Δ | Δ | 135 | 180 | Δ |
| Com. example 1 | Transfer molding | Yes | No | - | Yes | Yes | 350 | × | Δ | 135 | 180 | × |
| Com. example 2 | Cutter working | No | No | - | No | No | 500 | × | × | 130 | 180 | × |

As apparent from Table 1 above, with the working examples (present invention), good results were obtained. In contrast, with the comparative examples, satisfactory results were not obtained.

It is to be noted that, where EOG sterilization was carried out in place of the electron beam sterilization to carry out evaluation similar to that described above, similar results to those given above were obtained.

### Industrial Applicability

According to the present invention, when an elongated member is inserted into the valve body, the elongated member can be introduced into a portion of the first slit corresponding to the intersecting portion readily and with certainty by the first concave portion. Consequently, such a situation that any other portion
than the first slit or the second slit is pierced by the elongated member and the first slit or the second slit is damaged can be prevented. Further, since.the second concave portion is provided, the contact area of the inner faces of the first slit decreases, and consequently, the area over which blocking occurs when radiation sterilization is carried out (area over which the inner faces stick to each other) can be decreased. Further, when an elongated member is inserted into the valve body for the first time after sterilization, the second concave portion makes a start of exfoliating at the first slit which is in a sticking state. Consequently, upon insertion, exfoliating at the first slit and the second slit occurs readily, and damage to the first slit or the second slit can be prevented. Further, the penetration resistance upon insertion decreases, and the elongated member can be inserted readily into the valve body. Therefore, the valve body of the present invention can be used (installed) in a sheath of an introducer for introducing an elongated member for use with medical care such as, for example, a catheter or a guide wire into a living organism. Accordingly, the present invention has industrial applicability.

## Claims

1. A medical valve body (6) made of an elastic disk and having an opening and closing section (60) which opens and closes in response to insertion and pulling out of an elongated member (10) into and from said valve body (6), said valve body (6) having a first concave portion (64) formed on an upper face (601) side thereof and having a bottom face (641) formed as a concave face (641), said valve body (6) further having a lower face (602) on the opposite side to the upper face (601), wherein said opening and closing section (60) includes:
a first slit (61) extending to the concave face (641) but not to the lower face (602);
a second slit (62) extending to the lower face (602) but not to the concave face (641) and intersecting with the first slit (61) in the inside of said valve body (6), **characterized by**
a second concave portion (65) provided at a middle portion of the valve body (6) on the-concave face (641) at a location corresponding to an intersecting portion (63) between the first slit (61) and the second slit (62) as viewed in plan and formed, in the thickness direction of the valve body (6), to an intermediate portion of the first slit (61) but not extending to the second slit (62);
a pair of third concave portions (66) provided at the opposite end portions of said first slit (61) on the concave face side; the second concave portion (65), third concave portion (66) and first slit (61) communicating with each other and forming a single groove, such that by provision of the second and third concave portions, the contact area of the inner faces (612) of the first slit (61) is decreased, thereby facilitating exploitation and reducing penetration resistance upon insertion of an elongated member (10).

2. The valve body (6) according to claim 1, wherein said intersecting portion (63) is positioned at a central portion of said valve body (6) as viewed in plan, and
said first concave portion (64) includes said intersecting portion (63) as viewed in plan and said intersecting portion (63) is provided in such a manner as to be positioned at a central portion of said first concave portion (64) while
said first slit (61) is provided so as to extend from one end portion to the other end portion of said first concave portion (64).

3. The valve body (6) according to claim 1 or 2, wherein said first concave portion (64) has a bowl-like shape.

4. The valve body (6) according to any one of claims 1 to 3, wherein, in the unstressed state, the total value of the area of the third concave portions (66) as viewed in side elevation parallel to said first slit (61) is greater than the area of said second concave portion (65).

5. A medical tool (1) comprising a sheath (7) having a hub (2), sealing means (5), which is provided at an end portion of the hub (2) to seal the inside of the hub (2) and includes the valve body (6) according to any one of the preceding claims, and an elongated member (10) which is passed through the valve body (6),
said valve body (6) being installed such that the concave face (641) thereof is exposed outside a flow path in the hub (2) of the sheath (7), and the lower face (602) is exposed inside the flow path.

6. The medical tool according to claim 5,
wherein, in an unstressed state, a side face (652) in said second concave portion (65) is inclined by more than 45° with respect to a direction of movement of the elongated member (10) upon insertion and pulling off.

7. The medical tool according to claim 5 or 6,
wherein, in an unstressed state, a bottom face (651) in said second concave portion (65) is a flat face substantially perpendicular to a direction of movement of the elongated member (10) upon insertion and pulling off.

8. The medical tool according to any one of claims 5 to 7, further comprising
a mounting section on which the valve body (6) is mounted;
wherein, in a state wherein said valve body (6) is mounted on a mounting section, said second concave portion (65) is not closed.

9. The medical tool according to any one of claims 5 to 8, further comprising
a mounting section on which the valve body (6) is mounted;
wherein, in an unstressed state, said first slit (61) is open, and
said valve body (6) is deformed, when said valve body (6) is mounted on the mounting section, in such a manner as to be compressed toward said intersecting portion (63), and said first slit (61) is closed thereby.

10. The medical tool according to any one of claims 5 to 9, further comprising
a mounting section on which the valve body (6) is mounted;
wherein, in an unstressed state, said first slit (61) and said second slit (62) are open, and
said valve body (6) is deformed, when said valve body (6) is mounted on the mounting section, in such a manner as to be compressed toward said intersecting portion (63), and said first slit (61) is closed thereby.

11. The medical tool according to any one of claims 5 to 10,
wherein, in a state wherein said valve body (6) is mounted on a mounting section, said third concave portions (66) are not closed.

## Patentansprüche

1. Medizinischer Ventilkörper (6), der aus einer elastischen Scheibe besteht und einen Öffnungs- und Schließbereich (60) hat, der sich als Reaktion auf das Einfügen und Herausziehen eines länglichen Elements (10) in und aus dem Ventilkörper (6) öffnet und schließt, wobei der Ventilkörper (6) einen ersten konkaven Abschnitt (64) hat, der an einer Seite seiner oberen Fläche (601) ausgebildet ist und eine Bodenfläche (641) hat, die als eine konkave Fläche (641) ausgebildet ist, wobei der Ventilkörper (6) des Weiteren eine untere Fläche (602) an der zu der oberen Fläche (601) entgegengesetzten Seite hat, wobei der Öffnungs- und Schließbereich (60) Folgendes aufweist:
einen ersten Schlitz (61), der sich zu der konkaven Fläche (641) aber nicht zu der unteren Fläche (602) erstreckt;
einen zweiten Schlitz (62), der sich zu der unteren Fläche (602) aber nicht zu der konkaven Fläche (641) erstreckt und sich mit dem ersten Schlitz (61) im Inneren des Ventilkörpers (6) überschneidet,
**gekennzeichnet durch**
einen zweiten konkaven Abschnitt (65), der bei Betrachtung in einer Draufsicht an einem mittleren Abschnitt des Ventilkörpers (6) an der konkaven Fläche (641) an einem Ort entsprechend einem Überschneidungsabschnitt (63) zwischen dem ersten Schlitz (61) und dem zweiten Schlitz (62) vorgesehen ist und in der Dickenrichtung des Ventilkörpers (6) an einem Teilabschnitt des ersten Schlitz' (61) ausgebildet ist, sich aber nicht zu dem zweiten Schlitz (62) erstreckt;
ein Paar dritte konkave Abschnitte (66), die an den gegenüberliegenden Endabschnitten des ersten Schlitzes (61) an der Seite der konkaven Fläche vorgesehen sind; wobei der zweite konkave Abschnitt (65), der dritte konkave Abschnitt (66) und der erste Schlitz (61) miteinander in Verbindung sind und eine einzige Nut bilden, so dass **durch** das Vorsehen des zweiten und des dritten konkaven Abschnitts der Kontaktbereich der Innenflächen (612) des ersten Schlitzes (61) verkleinert wird, wodurch eine Delaminierung und eine Reduzierung des Durchdringungswiderstands beim Einfügen eines länglichen Elements (10) unterstützt werden.

2. Ventilkörper (6) gemäß Anspruch 1, wobei der Überschneidungsabschnitt (63) bei Betrachtung in einer Draufsicht an einem mittleren Abschnitt des Ventilkörpers (6) positioniert ist, und
der erste konkave Abschnitt (64) bei Betrachtung in der Draufsicht den Überschneidungsabschnitt (63) aufweist und der Überschneidungsabschnitt (63) derart vorgesehen ist, dass er an einem mittleren Abschnitt des ersten konkaven Abschnitts (64) positioniert ist, während
der erste Schlitz (61) so vorgesehen ist, dass er sich von einem Endabschnitt zu dem anderen Endabschnitt des ersten konkaven Abschnitts (64) erstreckt.

3. Ventilkörper (6) gemäß Anspruch 1 oder 2, wobei der erste konkave Abschnitt (64) eine schüsselartige Form hat.

4. Ventilkörper (6) gemäß einem der Ansprüche 1 bis 3, wobei im entspannten Zustand der Gesamtwert des Flächeninhalts des dritten konkaven Abschnitts (66) bei Betrachtung einer seitlichen Ansicht parallel zu dem ersten Schlitz (61) größer ist als der Flächeninhalt des zweiten konkaven Abschnitts (65).

5. Medizinisches Werkzeug (1), das eine Hülse (7) aufweist, die eine Nabe (2), eine Dichteinrichtung (5), die an einem Endabschnitt der Nabe (2) zum Abdichten des Inneren der Nabe (2) vorgesehen ist und den Ventilkörper (6) gemäß einem der vorherigen Ansprüche aufweist, und ein längliches Element (10) hat, das durch den Ventilkörper (6) hindurch tritt,
wobei der Ventilkörper (6) so installiert ist, dass seine konkave Fläche (641) außerhalb eines Strömungspfads in der Nabe (2) der Hülse (7) freiliegt und die untere Fläche (602) im Inneren des Strömungspfads freiliegt.

6. Medizinisches Werkzeug gemäß Anspruch 5,
wobei in einem entspannten Zustand eine Seitenfläche (652) in dem zweiten konkaven Abschnitt (65) um mehr als 45° hinsichtlich einer Bewegungsrichtung des länglichen Elements (10) beim Einfügen und Herausziehen geneigt ist.

7. Medizinisches Werkzeug gemäß Anspruch 5 oder 6,
wobei in einem entspannten Zustand eine Bodenfläche (651) in dem zweiten konkaven Abschnitt (65) eine ebene Fläche ist, die im Wesentlichen senkrecht zu einer Bewegungsrichtung des länglichen Elements (10) beim Einfügen und Herausziehen ist.

8. Medizinisches Werkzeug gemäß einem der Ansprüche 5 bis 7, des Weiteren mit,
einem Montagebereich, an dem der Ventilkörper (6) montiert ist;
wobei in einem Zustand, bei dem der Ventilkörper (6) an einem Montagebereich montiert ist, der zweite konkave Abschnitt (65) nicht geschlossen ist.

9. Medizinisches Werkzeug gemäß einem der Ansprüche 5 bis 8, des Weiteren mit,
einem Montagebereich, an dem der Ventilkörper (6) montiert ist;
wobei in einem entspannten Zustand der erste Schlitz (61) geöffnet ist, und
wobei der Ventilkörper (6) verformt wird, wenn der Ventilkörper (6) an den Montagebereich montiert wird, und zwar derart, dass er zu dem Überschneidungsabschnitt (63) hin komprimiert wird, und wobei der erste Schlitz (61) dadurch geschlossen wird.

10. Medizinisches Werkzeug gemäß einem der Ansprüche 5 bis 9, des Weiteren mit,
einem Montagebereich, an dem der Ventilkörper (6) montiert ist;
wobei in einem entspannten Zustand der erste Schlitz (61) und der zweite Schlitz (62) geöffnet sind, und
wobei der Ventilkörper (6) verformt wird, wenn der Ventilkörper (6) an den Montagebereich montiert wird, und zwar derart, dass er zu dem Überschneidungsabschnitt (63) hin komprimiert wird, und dass dadurch der erste Schlitz (61) geschlossen wird.

11. Medizinisches Werkzeug gemäß einem der Ansprüche 5 bis 10,
wobei in einem Zustand, bei dem der Ventilkörper (6) an einem Montagebereich montiert ist, die dritten konkaven Abschnitte (66) nicht geschlossen sind.

## Revendications

1. Corps de soupape médicale (6) réalisé à partir d'un disque élastique et ayant une section d'ouverture et de fermeture (60) qui s'ouvre et se ferme en réponse à l'insertion et au retrait d'un élément allongé (10) dans et dudit corps de soupape (6), ledit corps de soupape (6) ayant une première partie concave (64) formée sur son côté de face supérieure (601) et ayant une face de fond (641) formée comme une face concave (641), ledit corps de soupape (6) ayant en outre une face inférieure (602) sur le côté opposé à la face supérieure (601), dans lequel ladite section d'ouverture et de fermeture (60) comprend :
une première fente (61) s'étendant vers la face concave (641) mais pas vers la face inférieure (602) ;
une seconde fente (62) s'étendant vers la face inférieure (602) mais pas vers la face concave (641) et coupant la première fente (61) à l'intérieur dudit corps de soupape (6),
**caractérisé par** :
une deuxième partie concave (65) prévue au niveau d'une partie centrale du corps de soupape (6) sur la face concave (641) à un emplacement correspondant à une partie d'intersection (63) entre la première fente (61) et la seconde fente (62), comme observé en plan et formée, dans le sens de l'épaisseur du corps de soupape (6), sur une partie intermédiaire de la première fente (61) mais ne s'étendant pas vers la seconde fente (62) ;
une paire de troisièmes parties concaves (66) prévues au niveau des parties d'extrémité opposées de ladite première fente (61) sur le côté de face concave ; la deuxième partie concave (65), la troisième partie concave (66) et la première fente (61) communiquant entre elles et formant une rainure unique de sorte qu'en prévoyant les deuxième et troisièmes parties concaves, la zone de contact des faces internes (612) de la première fente (61) est réduite, facilitant ainsi l'exfoliation et réduisant la résistance à la pénétration suite à l'insertion d'un élément allongé (10).

2. Corps de soupape (6) selon la revendication 1, dans lequel ladite partie d'intersection (63) est positionnée au niveau d'une partie centrale dudit corps de soupape (6), comme observé en plan, et
ladite première partie concave (64) comprend ladite partie d'intersection (63), comme observé en plan et ladite partie d'intersection (63) est prévue afin d'être positionnée au niveau d'une partie centrale de ladite première partie concave (64), alors que :
ladite première fente (61) est prévue afin de s'étendre d'une partie d'extrémité à l'autre partie d'extrémité de la première partie concave (64).

3. Corps de soupape (6) selon la revendication 1 ou 2, dans lequel ladite première partie concave (64) a une forme de cuvette.

4. Corps de soupape (6) selon l'une quelconque des revendications 1 à 3, dans lequel, à l'état non tendu, la valeur totale de la surface des troisièmes parties concaves (66), comme observé sur une vue en élévation latérale parallèle à ladite première fente (61), est supérieure à la surface de ladite deuxième partie concave (65).

5. Instrument médical (1), comprenant une gaine (7) ayant un raccord (2), des moyens d'étanchéité (5) qui sont prévus au niveau d'une partie d'extrémité du raccord (2) pour réaliser l'étanchéité de l'intérieur du raccord (2) et comprennent un corps de soupape (6) selon l'une quelconque des revendications précédentes, et un élément allongé (10) qui passe à travers le corps de soupape (6),
ledit corps de soupape (6) étant installé de sorte que sa face concave (641) est exposée à l'extérieur d'une trajectoire d'écoulement dans le raccord (2) de la gaine (7), et la face inférieure (602) est exposée à l'intérieur de la trajectoire d'écoulement.

6. Instrument médical selon la revendication 5,
dans lequel, dans un état non tendu, une face latérale (652) dans ladite deuxième partie concave (65) est inclinée selon une valeur supérieure à 45° par rapport à une direction de déplacement de l'élément allongé (10) suite à l'insertion et au retrait.

7. Instrument médical selon la revendication 5 ou 6,
dans lequel, dans un état non tendu, une face de fond (651) dans ladite deuxième partie concave (65) est une face plate sensiblement perpendiculaire à une direction de déplacement de l'élément allongé (10) suite à l'insertion et au retrait.

8. Instrument médical selon l'une quelconque des revendications 5 à 7, comprenant en outre :
une section de montage sur laquelle le corps de soupape (6) est monté ;
dans lequel, dans un état dans lequel ledit corps de soupape (6) est monté sur une section de montage, ladite deuxième partie concave (65) n'est pas fermée.

9. Instrument médical selon l'une quelconque des revendications 5 à 8, comprenant en outre :
une section de montage sur laquelle le corps de soupape (6) est monté ;
dans lequel, dans un état non tendu, ladite première fente (61) est ouverte, et
ledit corps de soupape (6) est déformé, lorsque ledit corps de soupape (6) est monté sur la section de montage, afin d'être comprimé vers ladite partie d'intersection (63) et ladite première fente (61) est ainsi fermée.

10. Instrument médical selon l'une quelconque des revendications 5 à 9, comprenant en outre :
une section de montage sur laquelle le corps de soupape (6) est monté ;
dans lequel, dans un état non tendu, ladite première fente (61) et ladite seconde fente (62) sont ouvertes, et
ledit corps de soupape (6) est déformé, lorsque ledit corps de soupape (6) est monté sur la section de montage, afin d'être comprimé vers ladite partie d'intersection (63), et ladite première fente (61) est ainsi fermée.

11. Instrument médical selon l'une quelconque des revendications 5 à 10,
dans lequel, dans un état dans lequel ledit corps de soupape (6) est monté sur une section de montage, lesdites troisièmes parties concaves (66) ne sont pas fermées.
